# DEMANDE DE BREVET EUROPEEN

(11) **EP 1 231 277 A2**
(43) Date de publication de la demande: **14.08.2002**
(21) Numéro de dépôt: 02003797.4
(22) Date de dépôt: 21.04.1994
(51) Int. Cl.: C12N 15/86, A61K 48/00, C12N 5/10, A61L 27/00, A61K 9/14, C12N 9/24, C12N 15/27, C12N 9/12, C12N 15/12, C12N 15/56, C12N 15/54

(54) **Implant biocompatible pour la formation in vivo d'un neo-organe**

(30) Priorité: 21.04.1993 FR 9304700; 26.07.1993 FR 9309185
(62) Demande divisionnaire de: 94914431.5
(71) Demandeur: INSTITUT PASTEUR, 75724 Paris Cédex 15 (FR)
(72) Inventeur: Moullier, Philippe, 92100 Meudon (FR); Danos, Olivier, 92380 Garches (FR); Heard, Jean-Michel, 75014 Paris (FR); Ferry, Nicolas, 75013 Paris (FR)
(74) Mandataire: Desaix, Anne

(57) **Abrégé**

L'invention concerne un implant biocompatible pour le développement in vivo d'une structure comportant un tissu conjonctif. L'implant est caractérisé en ce qu'il comprend
- un support biocompatible permettant l'ancrage biologique de cellules ; et,
- des cellules eucaryotes ancrées dans et/ou à la surface dudit support biocompatible,
lesdites cellules eucaryotes ayant la capacité d'être tolérées immunologiquement par l'organisme dans lequel elles seraient susceptibles d'être implantées.

La présente invention concerne également les procédés de préparation de ces implants.

### Annex to the application documents - subsequently filed sequences listing

LISTE DE SEQUENCES

<110> INSTITUT PASTEUR

<120> IMPLANT BIOCOMPATIBLE POUR LA FORMATION IN VIVO D'UN
      NEO-ORGANE

<130> B2054ADA_01

<140> EP 02003797.4
<141> 2002-02-20

<150> EP 94914431.5
<151> 1994-04-21

<150> FR 9304700
<151> 1993-04-21

<150> FR 9309185
<151> 1993-07-26

<160> 1

<170> PatentIn Ver. 2.1

<210> 1
<211> 8388
<212> ADN
<213> Séquence artificielle

<220>
<223> Description de la séquence artificielle: vecteur
      rétroviral

<400> 1
tgaaagaccc cacctgtagg tttggcaagc tagcttaagt aacgccattt tgcaaggcat 60
ggaaaaatac ataactgaga atagagaagt tcagatcaag gtcaggaaca gatggaacag 120
ctgaatatgg gccaaacagg atatctgtgg taagcagttc ctgccccggc tcagggccaa 180
gaacagatgg aacagctgaa tatgggccaa acaggatatc tgtggtaagc agttcctgcc 240
ccggctcagg gccaagaaca gatggtcccc agatgcggtc cagccctcag cagtttctag 300
agaaccatca gatgtttcca gggtgcccca aggacctgaa atgaccctgt gccttatttg 360
aactaaccaa tcagttcgct tctcgcttct gttcgcgcgc ttctgctccc cgagctcaat 420
aaaagagccc acaacccctc actcggggcg ccagtcctcc gattgactga gtcgcccggg 480
tacccgtgta tccaataaac cctcttgcag ttgcatccga cttgtggtct cgctgttcct 540
tgggagggtc tcctctgagt gattgactac ccgtcagcgg gggtctttca tttgggggct 600
cgtccgggat cgggagaccc ctgcccaggg accaccgacc caccaccggg aggtaagctg 660
gccagcaact tatctgtgtc tgtccgattg tctagtgtct atgactgatt ttatgcgcct 720
gcgtcggtac tagttagcta actagctctg tatctggcgg acccgtggtg gaactgacga 780

gttcggaaca cccggccgca accctgggag acgtcccagg gacttcgggg gccgtttttg 840
tggcccgacc tgagtccaaa aatcccgatc gttttggact ctttggtgca ccccccttag 900
aggagggata tgtggttctg gtaggagacg agaacctaaa acagttcccg cctccgtctg 960
aatttttgct ttcggtttgg gaccgaagcc gcgccgcgcg tcttgtctgc tgcagcatcg 1020
ttctgtgttg tctctgtctg actgtgtttc tgtatttgtc tgagaatatg ggcccgcggg 1080
ccagactgtt accactccct taagtttgac cttaggtcac tggaaagatg tcgagcggat 1140
cgctcacaac cagtcggtag atgtcaagaa gagacgttgg gttaccttct gctctgcaga 1200
atggccaacc tttaacgtcg gatggccgcg agacggcacc tttaaccgag acctcatcac 1260
ccaggttaag atcaaggtct tttcacctgg cccgcatgga cacccagacc aggtccccta 1320
catcgtgacc tgggaagcct tggcttttga cccccctccc tgggtcaagc cctttgtaca 1380
ccctaagcct ccgcctcctc ttcctccatc cgccccgtct ctcccccttg aacctcctcg 1440
ttcgaccccg cctcgatcct ccctttatcc agccctcact ccttctctag gcgccaaacc 1500
taaacctcaa gttctttctg acagtggggg gccgctcatc gacctactta cagaagaccc 1560
cccgccttat agggacccaa gaccaccccc ttccgacagg gacggaaatg gtggagaagc 1620
gacccctgcg ggagaggcac cggacccctc cccaatggca tctcgcctac gtgggagacg 1680
ggagccccct gtggccgact ccactacctc gcaggcattc cccctccgcg caggaggaaa 1740
cggacagctt caatactggc cgttctcctc ttctgacctt tacaactgga aaaataataa 1800
cccttctttt tctgaagatc caggtaaact gacagctctg atcgagtctg ttctcatcac 1860
ccatcagccc acctgggacg actgtcagca gctgttgggg actctgctga ccggagaaga 1920
aaaacaacgg gtgctcttag aggctagaaa ggcggtgcgg ggcgatgatg ggcgccccac 1980
tcaactgccc aatgaagtcg atgccgcttt tcccctcgag aattctaccg ggtaggggag 2040
gcgcttttcc caaggcagtc tggagcatgc gctttagcag ccccgctggc acttggcgct 2100
acacaagtgg cctctggcct cgcacacatt ccacatccac cggtagcgcc aaccggctcc 2160
gttctttggt ggccccttcg cgccaccttc tactcctccc ctagtcagga agttcccccc 2220
gccccgcagc tcgcgtcgtg caggacgtga caaatggaag tagcacgtct cactagtctc 2280
gtgcagatgg acagcaccgc tgagcaatgg aagcgggtag gcctttgggg cagcggccaa 2340
tagcagcttt gctccttcgc tttctgggct cagaggctgg gaagggttgg gtccgggggc 2400
gggctcaggg gcgggctcag ggcgggggcg ggcgcgaagg tcctccggag cccggcattc 2460
tgcacgcttc aaaagcgcac gtctgccgcg ctgttctcct cttcctcatc tccgggcctt 2520
tcgaccggat ccggcgatta gtccaatttg ttaaagacag gatatcagtg gtccaggctc 2580
tagttttgac tcaacaatat caccagctga agcctataga gtacgagcca tagataaaat 2640
aaaagatttt atttagtctc cagaaaaagg ggggaatgaa agaccccacc tgtaggtttg 2700
gcaagctagc ttaagtaacg ccattttgca aggcatggaa aaatacataa ctgagaatag 2760
agaagttcag atcaaggtca ggaacagatg gaacagggtc gaccctagag aaccatcaga 2820
tgtttccagg gtgccccaag gacctgaaat gaccctgtgc cttatttgaa ctaaccaatc 2880
agttcgcttc tcgcttctgt tcgcgcgctt ctgctccccg agctcaataa aagagcccac 2940
aacccctcac tcggggcgcc agtcctccga ttgactgagt cgcccgggta cccgtgtatc 3000
caataaaccc tcttgcagtt gcatccgact tgtggtctcg ctgttccttg ggagggtctc 3060
ctctgagtga ttgactaccc gtcagcgggg gtctttcatt tatgtgtcat aaatatttct 3120
aattttaaga tagtatctcc attggctttc tactttttct ttttattttt ttttgtcctc 3180
tgtctccatg tgttgttgtt gttgtttgtt tgtttgtttg ttggttggtt ggttaatttt 3240
tttttaaaga tcctacacta tagttcaagc tagactatta gctactctgt aacccagggt 3300
gaccttgaag tcatgggtag cctgctgttt tagccttccc acatctaaga ttacaggtat 3360
gagctatcat tttggtatat tgattgattg attgattgat gtgtgtgtgt gtgattgtgt 3420
ttgtgtgtgt gattgtgtat atgtgtgtat ggttgtgtgt gattgtgtgt atgtatgttt 3480
gtgtgtgatt gtgtgtgtgt gattgtgcat gtgtgtgtgt gatgtgttag tgtatgattg 3540
tgtgtgtgtg tgtgtgtgtg tgtgtgtgtg tgtgtgtgtg tgtgtgttgt gtatatatat 3600
ttatggtagt gagaggcaac gctccggccc aggcgtcagg ttggtttttg agacagagtc 3660

tttcacttag cttgaattct tgaagacgaa agggcctcgt gatacgccta tttttatagg 3720
ttaatgtcat gataataatg gtttcttaga cgtcaggtgg cacttttcgg ggaaatgtgc 3780
gcggaacccc tatttgttta tttttctaaa tacattcaaa tatgtatccg ctcatgagac 3840
aataaccctg ataaatgctt caataatatt gaaaaaggaa gagtatgagt attcaacatt 3900
tccgtgtcgc ccttattccc ttttttgcgg cattttgcct tcctgttttt gctcacccag 3960
aaacgctggt gaaagtaaaa gatgctgaag atcagttggg tgcacgagtg ggttacatcg 4020
aactggatct caacagcggt aagatccttg agagttttcg ccccgaagaa cgttttccaa 4080
tgatgagcac ttttaaagtt ctgctatgtg gcgcggtatt atcccgtgtt gacgccgggc 4140
aagagcaact cggtcgccgc atacactatt ctcagaatga cttggttgag tactcaccag 4200
tcacagaaaa gcatcttacg gatggcatga cagtaagaga attatgcagt gctgccataa 4260
ccatgagtga taacactgcg gccaacttac ttctgacaac gatcggagga ccgaaggagc 4320
taaccgcttt tttgcacaac atgggggatc atgtaactcg ccttgatcgt tgggaaccgg 4380
agctgaatga agccatacca aacgacgagc gtgacaccac gatgcctgca gcaatggcaa 4440
caacgttgcg caaactatta actggcgaac tacttactct agcttcccgg caacaattaa 4500
tagactggat ggaggcggat aaagttgcag gaccacttct gcgctcggcc cttccggctg 4560
gctggtttat tgctgataaa tctggagccg gtgagcgtgg gtctcgcggt atcattgcag 4620
cactggggcc agatggtaag ccctcccgta tcgtagttat ctacacgacg gggagtcagg 4680
caactatgga tgaacgaaat agacagatcg ctgagatagg tgcctcactg attaagcatt 4740
ggtaactgtc agaccaagtt tactcatata tactttagat tgatttaaaa cttcattttt 4800
aatttaaaag gatctaggtg aagatccttt ttgataatct catgaccaaa atcccttaac 4860
gtgagttttc gttccactga gcgtcagacc ccgtagaaaa gatcaaagga tcttcttgag 4920
atcctttttt tctgcgcgta atctgctgct tgcaaacaaa aaaaccaccg ctaccagcgg 4980
tggtttgttt gccggatcaa gagctaccaa ctctttttcc gaaggtaact ggcttcagca 5040
gagcgcagat accaaatact gtccttctag tgtagccgta gttaggccac cacttcaaga 5100
actctgtagc accgcctaca tacctcgctc tgctaatcct gttaccagtg gctgctgcca 5160
gtggcgataa gtcgtgtctt accgggttgg actcaagacg atagttaccg gataaggcgc 5220
agcggtcggg ctgaacgggg ggttcgtgca cacagcccag cttggagcga acgacctaca 5280
ccgaactgag atacctacag cgtgagctat gagaaagcgc cacgcttccc gaagggagaa 5340
aggcggacag gtatccggta agcggcaggg tcggaacagg agagcgcacg agggagcttc 5400
cagggggaaa cgcctggtat ctttatagtc ctgtcgggtt tcgccacctc tgacttgagc 5460
gtcgattttt gtgatgctcg tcaggggggc ggagcctatg gaaaaacgcc agcaacgcgg 5520
cctttttacg gttcctggcc ttttgctggc cttttgctca catgttcttt cctgcgttat 5580
cccctgattc tgtggataac cgtattaccg cctttgagtg agctgatacc gctcgccgca 5640
gccgaacgac cgagcgcagc gagtcagtga gcgaggaagc ggaagagcgc ctgatgcggt 5700
attttctcct tacgcatctg tgcggtattt cacaccgcat atggtgcact ctcagtacaa 5760
tctgctctga tgccgcatag ttaagccagt atacactccg ctatcgctac gtgactgggt 5820
catggctgcg ccccgacacc cgccaacacc cgctgacgcg ccctgacggg cttgtctgct 5880
cccggcatcc gcttacagac aagctgtgac cgtctccggg agctgcatgt gtcagaggtt 5940
ttcaccgtca tcaccgaaac gcgcgaggca gctgcggtaa agctcatcag cgtggtcgtg 6000
aagcgattca cagatgtctg cctgttcatc cgcgtccagc tcgttgagtt tctccagaag 6060
cgttaatgtc tggcttctga taaagcgggc catgttaagg gcggtttttt cctgtttggt 6120
cactgatgcc tccgtgtaag ggggatttct gttcatgggg gtaatgatac cgatgaaacg 6180
agagaggatg ctcacgatac gggttactga tgatgaacat gcccggttac tggaacgttg 6240
tgagggtaaa caactggcgg tatggatgcg gcgggaccag agaaaaatca ctcagggtca 6300
atgccagcgc ttcgttaata cagatgtagg tgttccacag ggtagccagc agcatcctgc 6360
gatgcagatc cggaacataa tggtgcaggg cgctgacttc cgcgtttcca gactttacga 6420
aacacggaaa ccgaagacca ttcatgttgt tgctcaggtc gcagacgttt tgcagcagca 6480
gtcgcttcac gttcgctcgc gtatcggtga ttcattctgc taaccagtaa ggcaaccccg 6540

ccagcctagc cgggtcctca acgacaggag cacgatcatg cgcacccgtg gccaggaccc 6600
aacgctgccc gagatgcgcc gcgtgcggct gctggagatg gcggacgcga tggatatgtt 6660
ctgccaaggg ttggtttgcg cattcacagt tctccgcaag aattgattgg ctccaattct 6720
tggagtggtg aatccgttag cgaggtgccg ccggcttcca ttcaggtcga ggtggcccgg 6780
ctccatgcac cgcgacgcaa cgcggggagg cagacaaggt atagggcggc gcctacaatc 6840
catgccaacc cgttccatgt gctcgccgag gcggcataaa tcgccgtgac gatcagcggt 6900
ccagtgatcg aagttaggct ggtaagagcc gcgagcgatc cttgaagctg tccctgatgg 6960
tcgtcatcta cctgcctgga cagcatggcc tgcaacgcgg gcatcccgat gccgccggaa 7020
gcgagaagaa tcataatggg gaaggccatc cagcctcgcg tcgcgaacgc cagcaagacg 7080
tagcccagcg cgtcggccgc catgccggcg ataatggcct gcttctcgcc gaaacgtttg 7140
gtggcgggac cagtgacgaa ggcttgagcg agggcgtgca agattccgaa taccgcaagc 7200
gacaggccga tcatcgtcgc gctccagcga aagcggtcct cgccgaaaat gacccagagc 7260
gctgccggca cctgtcctac gagttgcatg ataaagaaga cagtcataag tgcggcgacg 7320
atagtcatgc cccgcgccca ccggaaggag ctgactgggt tgaaggctct caagggcatc 7380
ggtcgacgct ctcccttatg cgactcctgc attaggaagc agcccagtag taggttgagg 7440
ccgttgagca ccgccgccgc aaggaatggt gcatgcaagg agatggcgcc caacagtccc 7500
ccggccacgg ggcctgccac catacccacg ccgaaacaag cgctcatgag cccgaagtgg 7560
cgagcccgat cttccccatc ggtgatgtcg gcgatatagg cgccagcaac cgcacctgtg 7620
gcgccggtga tgccggccac gatgcgtccg gcgtagagcg ccacaggacg ggtgtggtcg 7680
ccatgatcgc gtagtcgata gtggctccaa gtagcgaagc gagcaggact gggcggcggc 7740
caaagcggtc ggacagtgct ccgagaacgg gtgcgcatag aaattgcatc aacgcatata 7800
gcgctagcag cacgccatag tgactggcga tgctgtcgga atggacgata tcccgcaaga 7860
ggcccggcag taccggcata accaagccta tgcctacagc atccagggtg acggtgccga 7920
ggatgacgat gagcgcattg ttagatttca tacacggtgc ctgactgcgt tagcaattta 7980
actgtgataa actaccgcat taaagcttgg cttaggagtt tcctaataca tcccaaactc 8040
aaatatataa gcatttgact tgttctatgc cctaggggga ggggggaagc taagccagct 8100
ttttttaaca tttaaaatgt taattccatt ttaaatgcac agatgttttt atttcataag 8160
ggtttcaatg tgcatgaatg tcgcaatatc ctgttaccaa agctagtata aataaaaata 8220
gataaacgtg gaaattactt agagtttctg tcattaacgt ttccttcctc agttgacaac 8280
ataaatgcgc tgctgagaag ccagtttgca tctgtcagga tcaatttcca ttatgccagt 8340
catattaatt actagtcaat tagttgattt ttgacatata catgtgaa 8388

## Description

L'invention concerne un implant biocompatible pour l'expression chez l'homme ou chez l'animal de substances déterminées, notamment pour l'ancrage des cellules recombinantes de l'invention.

La présente invention concerne également des vecteurs rétroviraux pour la préparation de cellules recombinantes susceptibles d'être implantées in vivo dans un but thérapeutique.

### Etat de la technique

L'introduction in vivo d'implants susceptibles d'exprimer des substances déterminées, par exemple à des fins thérapeutiques, nécessite l'utilisation de moyens efficaces s'agissant de la finalité thérapeutique ou prophylactique recherchée et la capacité pour l'organisme chez lequel est introduit l'implant, de le tolérer à plus ou moins long terme.

La demande de brevet internationale antérieure publiée sous le numéro 92/15676, décrit des moyens pour réaliser in vivo l'expression de séquences de nucléotides déterminées, en vue d'un traitement thérapeutique de maladies résultant d'une anomalie génétique. Cette demande internationale 92/15676, propose d'utiliser des fibroblastes génétiquement modifiés par un vecteur rétroviral, en vue de les implanter dans le tissu conjonctif de la peau du sujet à traiter.

La séquence de nucléotides dont l'expression est recherchée dans cette demande internationale antérieure, est placée sous le contrôle de la séquence LTR (Long Terminal Repeat) du vecteur rétroviral et/ou sous le contrôle de promoteurs exogènes constitutifs ou inductibles, la séquence LTR du rétrovirus étant néanmoins conservée lorsque le promoteur exogène est présent.

### Résumé de l'invention

L'invention propose des moyens permettant de réaliser in vivo, l'expression d'une séquence de nucléotides choisie, dans des conditions telles que cette expression est obtenue sur une durée de plusieurs mois, de préférence plus de 6 mois, de telle façon que le produit de la séquence de nucléotides exprimée est présent en quantité suffisante et dans des conditions appropriées pour produire un effet thérapeutique recherché in vivo.

Les moyens définis dans la présente demande permettent de réaliser l'expression et la sécrétion d'une protéine, glycoprotéine, ou d'un peptide ayant un intérêt biologique, pour autoriser son utilisation thérapeutique in vivo.

L'invention concerne également l'utilisation de matériaux biocompatibles, en tant que supports pour l'introduction chez l'homme ou chez l'animal, de cellules, par exemple de cellules recombinantes de l'invention définies ci-dessous, à partir desquelles on souhaite produire une molécule déterminée, notamment à des fins thérapeutiques.

La préparation d'implants fait appel à de tels supports, propres à être mis au contact de cellules et de différents facteurs favorisant l'adhérence de ces cellules au support si nécessaire, dans des conditions telles que les différents constituants présents conservent leurs propriétés fonctionnelles et structurales naturelles principales.

Ces supports biocompatibles, d'origine biologique ou non peuvent être résorbables ou non résorbables par l'hôte chez lequel ils sont introduits.

L'invention concerne également des vecteurs rétroviraux ayant la capacité d'infecter des cellules et en particulier des cellules eucaryotes ainsi que les cellules recombinantes comprenant ces vecteurs. Les cellules recombinantes de l'invention peuvent être administrées, ou implantées chez un patient, et produisant ainsi in vivo une protéine ou tout produit d'expression d'une séquence de nucléotides déterminée, insérée dans vecteur rétroviral.

Les inventeurs ont examiné quels étaient les paramètres qui, à différents niveaux, permettraient d'exprimer in vivo une séquence de nucléotides déterminée, de façon à améliorer l'expression de la séquence de nucléotides insérée dans le vecteur afin d'obtenir un effet thérapeutique, si nécessaire de longue durée.

Ainsi, ils ont mis en évidence que lorsque la séquence de nucléotides exogène dont on cherche l'expression est avantageusement placée dans le vecteur destiné à l'infection des cellules, sous le contrôle d'un promoteur exogène constitutif ou inductible, la séquence LTR interne au vecteur rétroviral doit alors être partiellement supprimée. La délétion doit être suffisante pour affecter la transcription de l'ARNm.

### Description détaillée de l'invention

### Implant biocompatible

L'implant ou néo-organe de l'invention est obtenu par le procédé qui consiste en l'assemblage in vitro de différents éléments afin de former un implant qui est introduit de préférence dans la cavité péritonéale du receveur. D'autres sites d'implantation sont utilisables tels que l'espace péri-rénal, le derme. In vivo, l'implant donne naissance à un tissu conjonctif néo-formé, vascularisé, non modifié au cours du temps.

De façon générale, l'implant de l'invention est caractérisé en ce qu'il comprend :
- un support biocompatible permettant l'ancrage biologique de cellules ;
- des cellules ayant la capacité d'exprimer et de secréter naturellement ou après recombination un composé déterminé, par exemple un composé ayant un intérêt thérapeutique ; et
- un constituant capable d'induire et/ou de favoriser la gélification desdites cellules.

Particulièrement, les éléments participant à l'assemblage de l'implant in vitro sont les suivants:
1) **Un support rigide.** Il peut être préparé à partir de différents biomatériaux: PTFE, corail, fibres de collagène réticulé ;
2) **Un gel de collagène.** On peut utiliser du collagène de queue de rat, du collagène bovin, du collagène humain ; et
3) **des cellules génétiquement modifiées,** de préférence les cellules recombinantes de la présente invention, qui sont décrites de façon détaillée plus loin.

L'invention a donc pour objet un implant ou néo-organe caractérisé en ce qu'il comprend un support rigide biocompatible, particulièrement un support rigide de PTFE ou d'origine biologique, permettant l'ancrage biologique de cellules mises en contact, préalablement ou non avec des constituants capables d'induire et/ou de favoriser leur inclusion au sein d'une matrice formant un gel, lesdites cellules étant choisies pour leur capacité à exprimer et à sécréter naturellement ou après recombinaison un composé déterminé, par exemple un composé ayant un intérêt thérapeutique.

L'expression "ancrage biologique" signifie que les cellules contenues dans l'implant peuvent se fixer à la surface du support biocompatible ou, dans certains cas, pénétrer à l'intérieur de ce support.

Cette fixation des cellules sur le support est permise notamment par la présence des constituants capables d'induire et/ou de favoriser l'inclusion des cellules au sein d'une matrice ayant la constitution d'un gel.

Cette inclusion dans la matrice, appelée gélification permet l'organisation des cellules en une structure à trois dimensions dans un environnement amorphe, ne donnant pas lieu in vivo à une inflammation prolongée.

L'implant obtenu est selon un premier mode de réalisation de l'invention constitué d'une part d'un support biocompatible, tel qu'un support comprenant un matériau biocompatible synthétique, notamment des fibres de polytétrafluoroéthylène (PTFE) ou un support comprenant un matériau d'origine biologique à base de calcaire, en particulier à base de carbonate de calcium et, de préférence de corail et d'autre part d'un gel éventuellement chargé de cellules exprimant la substance d'intérêt, notamment de cellules recombinantes.

L'invention concerne également une méthode de préparation d'un implant. La méthode comprend les étapes de :
- mise en contact du support biocompatible avec lesdites cellules et un constituant capable d'induire et/ou de favoriser leur gélification ;
- incubation de la préparation obtenue à l'étape précédente afin d'obtenir la gélification desdits constituants ;
- culture des cellules ainsi obtenues dans des conditions leur permettant de s'attacher aux constituants gélifiés, et
- récupération de l'implant ainsi obtenu.

### a) Implant comprenant un matériau biocompatible synthétique.

L'invention a pour objet un implant ou néo-organe caractérisé en ce qu'il comprend des cellules exprimant la substance d'intérêt, en combinaison avec des fibres de polytétrafluoroéthylène (PTFE) et du collagène. Un tel implant est susceptible lorsqu'introduit in vivo, de constituer un néo-organe vascularisé, individualisé au sein d'un tissu.

Avantageusement, l'implant tel que décrit ci-dessus comprend en outre un facteur de croissance, par exemple le bFGF(basic Fibroblast Growth Factor). Ce facteur de croissance favorise la vascularisation du néo-organe lorsque celui-ci est implanté in vivo.

De préférence, le néo-organe est introduit dans la cavité péritonéale chez un patient chez lequel on souhaite obtenir l'expression de la séquence de nucléotides exogène contenue dans le vecteur de l'invention. Cette localisation du néo-organe est favorable au développement de sa vascularisation et au maintien sous forme invididualisée de cet organe. L'implantation du néo-organe comprenant les cellules recombinantes selon l'invention peut être permanente, ou au contraire transitoire, le néo-organe formé pouvant dans ce cas être retiré après un temps donné d'implantation.

L'invention vise par ailleurs un procédé pour la préparation d'un implant ci-dessus défini, comprenant les étapes de :
- mise en contact de cellules recombinantes avec une solution de collagène et des fibres de PTFE de préférence préalablement traitées avec du collagène et optionnellement avec un facteur de croissance angiogénique,
- incubation de la préparation obtenue à l'étape précédente afin d'obtenir la gélification du collagène,
- culture des cellules ainsi obtenues pendant un temps suffisant pour permettre aux cellules de s'attacher sur les fibres de collagène,
- récupération de l'implant ainsi obtenu.

### b) Implant comprenant un matériau biocompatible d'origine biologique.

De préférence, le matériau utilisé est un corail à haute porosité, c'est-à-dire qu'il possède une porosité telle que décrite dans le brevet français n° 2 460 657. Il peut s'agir aussi d'une poudre de porosité suffisante pour permettre d'obtenir une armature solide en vue de la constitution d'un réseau cellulaire comportant ou non un support biologique tel que le collagène. Dans le cas où le corail est remplacé par du collagène, réticulé ou non, ce dernier peut servir également à la fois comme support et comme matrice favorisant la gélification biologique pour la constitution du réseau de cellules.

Selon un mode de réalisation avantageux de l'invention, le corail à haute porosité utilisé est de type sphérique.

Selon un autre mode de réalisation de l'invention, l'implant comporte un support d'origine biologique formant une matrice solide, ledit support contenant :
- du collagène plus ou moins réticulé par exemple sous forme de fibres ou d'éponges telles que commercialisées sous les marques Hémostagène® ou Paroguide® ;
- de l'os en poudre ou en fragment ou ;
- des polymères à base d'hydrates de carbones tels que le dextran ou l'acide hyaluronique.

Selon un mode de réalisation avantageux de l'invention, l'armature solide du support d'origine biologique est susceptible de résorption in vivo.

L'implant ainsi constitué permet la formation in vivo d'un néo-organe individualisé et stable si nécessaire, dans lequel le support biologique est susceptible d'être progressivement résorbé en laissant place à une structure comportant un tissu conjonctif vascularisé de forme relativement comparable à celle de l'implant, ce néo-organe ayant cependant le cas échéant un volume diminué par rapport au volume de l'implant d'origine.

Un implant particulièrement préféré dans le cadre de l'invention est tel que les constituants capables d'induire et/ou de favoriser la gélification des cellules sont par exemple du collagène non réticulé ou des alginates.

En particulier, on aura recours à du collagène par exemple de type I, notamment à une concentration de 1,5 mg/ml.

D'autres constituants pour gélifier les cellules peuvent être naturellement utilisés dès lors qu'ils ont la propriété fonctionnelle recherchée et qu'ils présentent les propriétés de biocompatibilité requises pour être introduits in vivo chez l'homme ou chez l'animal.

### c) Utilisation de l'implant de l'invention

Un implant particulier propre à la réalisation de l'invention est en outre caractérisé en ce que les cellules sont des cellules recombinées comportant une information génétique étrangère à leur génome, susceptibles d'exprimer cette information supplémentaire in vivo et ayant la capacité d'être tolérées immunologiquement par un organisme auquel elles seraient administrées.

De manière préférée, un implant selon l'invention est caractérisé en ce que les cellules recombinantes sont modifiées par un vecteur contenant une séquence ou plusieurs séquences de nucléotides exogène(s), codant pour un antigène ou un déterminant antigénique ou codant pour un polypeptide ou une glycoprotéine, soluble dans le sérum, par exemple pour un polypeptide ou une glycoprotéine d'intérêt thérapeutique, notamment pour une hormone, une protéine ou une glycoprotéine de structure ou une protéine ou une glycoprotéine du métabolisme ou une protéine ou une glycoprotéine virale ou une protéine ayant les caractéristiques d'un anticorps ou d'un fragment d'anticorps.

De façon préférée, l'implant contient en outre un ou plusieurs facteurs angiogéniques, en particulier du bFGF, préférablement incorporés lors de la mise en contact du support biocompatible avec les cellules et le constituant capable d'induire et/ou de favoriser leur gélification.

La fixation du facteur angiogénique, par exemple du bFGF, est favorisée par la présence de l'agent de gélification.

Cet agent contribue à la fixation stable des facteurs angiogéniques au support d'origine biologique de l'implant.

De façon particulièrement avantageuse, l'implant contient également de l'héparine ou un dérivé héparinique tel que l'héparan sulfate ou des fractions hépariniques.

L'héparine et ses dérivés sont capables de se fixer aux constituants favorisant la gélification et ainsi d'augmenter l'affinité des facteurs angiogéniques aux constituants permettant la gélification. En particulier, l'héparine se fixe au collagène et augmente ainsi l'affinité du bFGF pour le collagène.

L'implant selon l'invention peut être mis en oeuvre de façon permanente ou transitoire, pour l'utilisation chez l'homme ou chez l'animal. En effet, sa capacité à constituer in vivo un néo-organe individualisé, permet de le retirer par prélèvement si nécessaire.

Un implant selon l'invention est, de façon particulièrement intéressante, utilisable pour :
- le traitement de maladies génétiques, en particulier pour le traitement de maladies de surcharge lysosomale, de l'hémophilie A ou de l'hémophilie B, de la β-thalassémie, la séquence de nucléotides exogène contenue dans les cellules recombinantes correspondant respectivement à celles qui codent pour la β-glucuronidase pour le facteur VIII, le facteur IX ou l'érythropoïétine, ou pour une partie active de ces séquences ;
- le traitement de maladies acquises, par exemple pour le traitement de maladies virales, notamment pour le traitement d'une infection due au rétrovirus HIV par exemple par l'expression et la sécrétion dans le sérum de molécules CD4 solubles ou d'une protéine anti-virale soluble ;
- le traitement de tumeurs, la séquence de nucléotides exogène contenue dans les cellules recombinantes codant pour un composé capable de favoriser ou d'augmenter la réponse immunitaire contre les cellules de la tumeur.

L'invention a aussi pour objet une composition caractérisée en ce qu'elle comprend un implant selon l'invention et un ou plusieurs autres constituants tels que par exemple un antigène, un adjuvant notamment pour la vaccination.

Les moyens décrits dans l'invention permettent donc d'envisager le traitement de maladies génétiques ou acquises sur une période longue, supérieure à plusieurs mois, et ce sans administration répétée du produit d'expression de la séquence de nucléotides exogène dont on recherche l'activité thérapeutique. De plus, les moyens décrits dans l'invention permettent l'obtention sous forme de protéine sécrétée d'une quantité de produit d'expression de la séquence de nucléotides exogène, suffisante pour avoir un effet thérapeutique in vivo.

Une autre application des cellules recombinantes selon l'invention ou des compositions ou implants les contenant, concerne la préparation d'anticorps contre le produit d'expression de la séquence de nucléotides exogène, contenu dans le vecteur introduit dans les cellules recombinantes. De préférence, ces anticorps sont obtenus in vivo lorsque les cellules recombinantes sont implantées dans un organisme.

Cette utilisation permet par exemple de produire des anticorps contre un antigène dont on n'aurait pas identifié la séquence en acides aminés ni nécessairement séquencé l'ADN ou l'ADNc. Selon l'effet recherché, l'introduction du vecteur recombinant selon l'invention, sera effectuée dans un type cellulaire défini.

De même, l'invention permet la production in vivo d'anticorps contre un antigène déterminé, par exemple à des fins de vaccination d'un patient.

Des cellules recombinantes telles que décrites plus bas, ou des compositions ou implants les contenant peuvent également être mises en oeuvre pour le traitement de tumeurs, la séquence de nucléotides exogène contenue dans les cellules recombinantes codant pour une substance capable de favoriser ou d'augmenter la réponse immunitaire contre les cellules de la tumeur.

A titre particulier, les cellules recombinantes utilisables pour le traitement de tumeurs, peuvent être des cellules obtenues par recombinaison de cellules de tumeurs, prélevées chez un patient, avec un vecteur rétroviral répondant aux définitions données dans les pages suivantes.

### Kit selon l'invention

L'invention comprend également un kit pour la préparation d'un implant pour réaliser in vivo l'expression et la secrétion par des cellules d'un composé pour produire un effet thérapeutique recherché. Le kit de l'invention comprend généralement :
- un support biocompatible permettant l'ancrage biologique desdites cellules ; et
- un constituant capable d'induire et/ou de favoriser la gélification desdites cellules.

Particulièrement, le support biocompatible comprend au moins un des éléments choisis parmi le groupe comprenant le PTFE, ou un support d'origine biologique, particulièrement un support d'origine biologique à base de calcaire, en particulier de carbonate de calcium, de préférence de corail. Le constituant capable d'induire et/ou de favoriser la gélification des cellules est de préférence du collagène, en particulier du collagène de type I, de préférence à une concentration de l'ordre de 1,5 mg/ml.

Le kit selon l'invention peut également inclure un ADN comprenant une séquence codant pour le composé exprimé et secrété par lesdites cellules. Cet ADN peut être utilisé pour transformer les cellules prélevées chez le patient à traiter.

Plus particulièrement, l'ADN faisant partie du kit est un vecteur rétroviral tel que décrit plus loin.

Le kit peut aussi comprendre des cellules ayant la capacité d'exprimer et de secréter naturellement ou après recombinaison un composé déterminé, par exemple un composé ayant un. intérêt thérapeutique.

### Vecteur rétroviral et cellules recombinantes

### a) Vecteur rétroviral

Un nouveau vecteur rétroviral recombinant selon l'invention est caractérisé en ce qu'il comprend :
. une séquence d'ADN de provirus modifiée en ce que :
   - les gènes gag, pol et env ont été délétés au moins en partie et ce afin d'obtenir un ADN proviral incapable de se répliquer, cet ADN ne pouvant, en outre, être recombiné pour former un virus sauvage ;
   - la séquence LTR comporte une délétion dans la séquence U3, telle que la transcription sous forme d'ARNm qu'elle contrôle est réduite de manière significative, par exemple d'au moins 10 fois, et
   - ce vecteur rétroviral recombinant comprenant, en outre, une séquence de nucléotides exogène sous le contrôle d'un promoteur, par exemple d'un promoteur constitutif ou inductible, exogène.

Les éléments principaux formant ce vecteur sont décrits ci-dessous de façon détaillée.

### 1) ADN de provirus

Une séquence d'ADN de provirus ou "séquence d'ADN proviral" est une séquence d'ADN transcrite à partir de l'ARN génomique du virus lorsqu'il est intégré dans les cellules hôtes du virus. L'ADN du provirus comprend ainsi des séquences codant pour les protéines gag, pol et env du rétrovirus, qui correspondent respectivement aux nucléoprotéines, polymérases, protéines et glycoprotéines d'enveloppe.

Le vecteur rétroviral selon l'invention est tel que les gènes gag, pol et env de l'ADN du provirus ont été délétés au moins en partie et ce afin d'obtenir un ADN proviral incapable de se répliquer, cet ADN ne pouvant, en outre, être muté de façon réverse ou recombiné pour former un virus sauvage.

L'ADN du provirus comporte également des enchaînements appelés LTR (Long Terminal Repeat), qui comportent des régions appelées R, U3 et U5. Ces séquences de la région LTR interviennent dans le cycle de réplication du rétrovirus.

La séquence LTR de l'ADN proviral a également été mutée par délétion, par exemple dans sa partie U3 ; cette délétion affecte les fonctions de l'activateur interne de la région LTR. De plus, cette délétion permet de diminuer et de préférence, de neutraliser l'effet transcriptionnel de la région LTR, en conservant la capacité du promoteur exogène de promouvoir et contrôler l'expression de la séquence de nucléotides exogène contenue dans le vecteur rétroviral.

On peut aussi envisager la délétion de l'ensemble de la séquence constituant le promoteur et l'activateur. Toutefois, une telle délétion présente l'inconvénient d'être accompagnée d'une diminution importante des titres du vecteur.

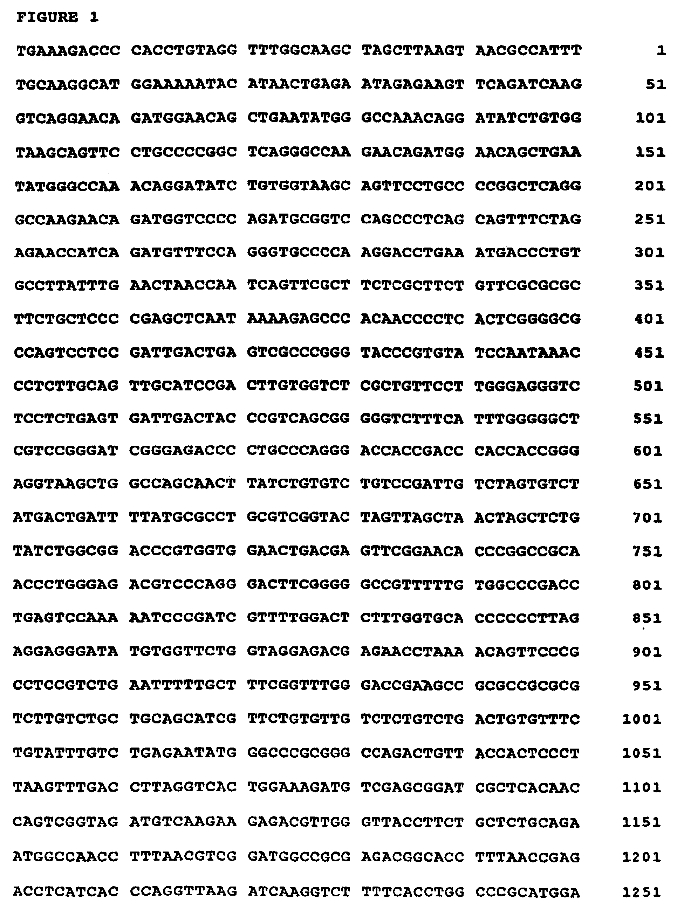

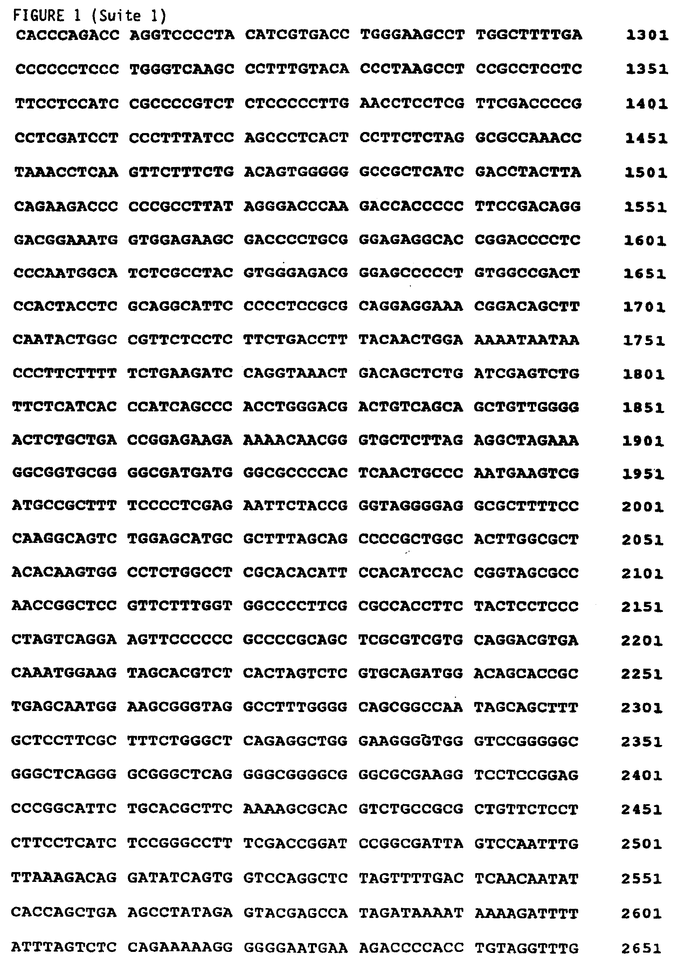

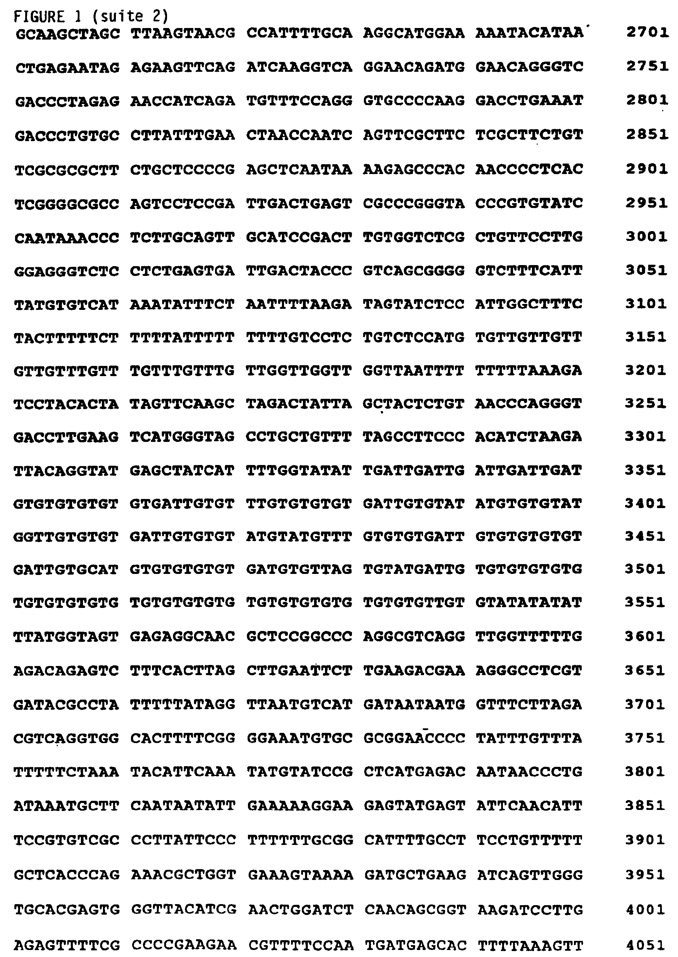

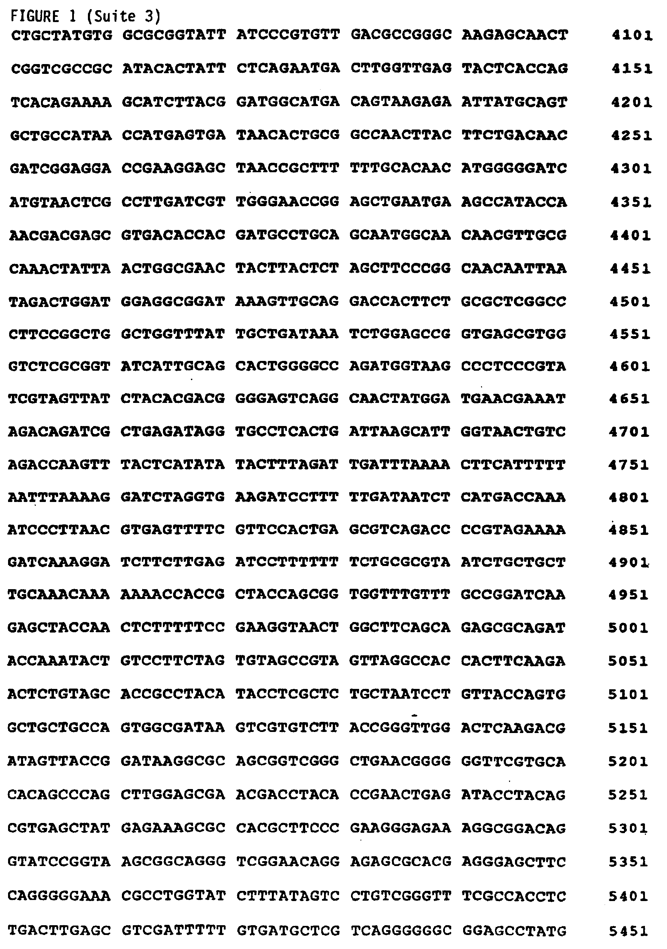

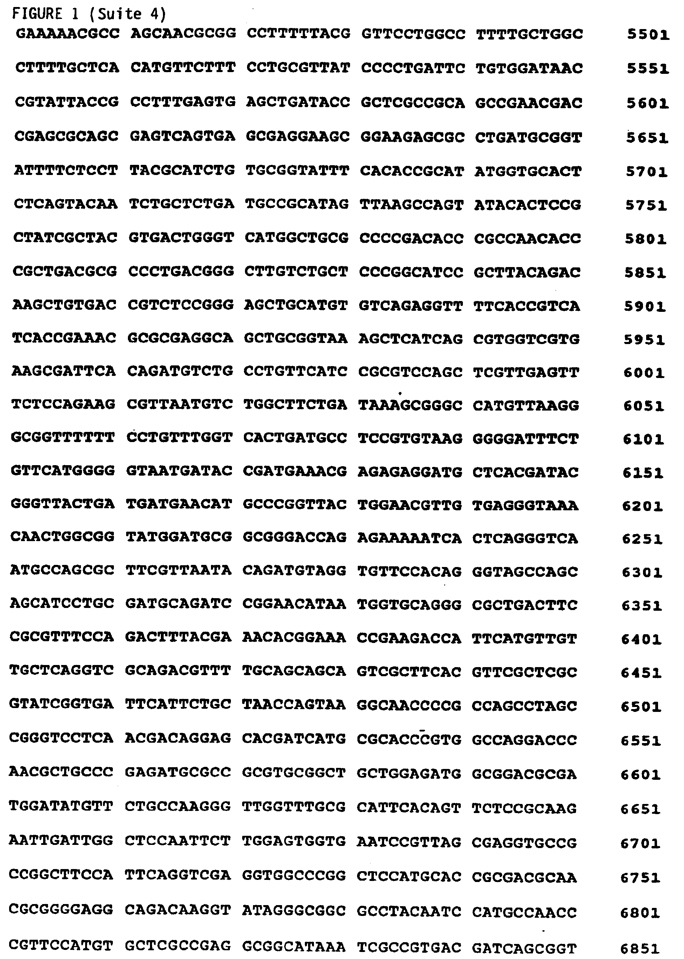

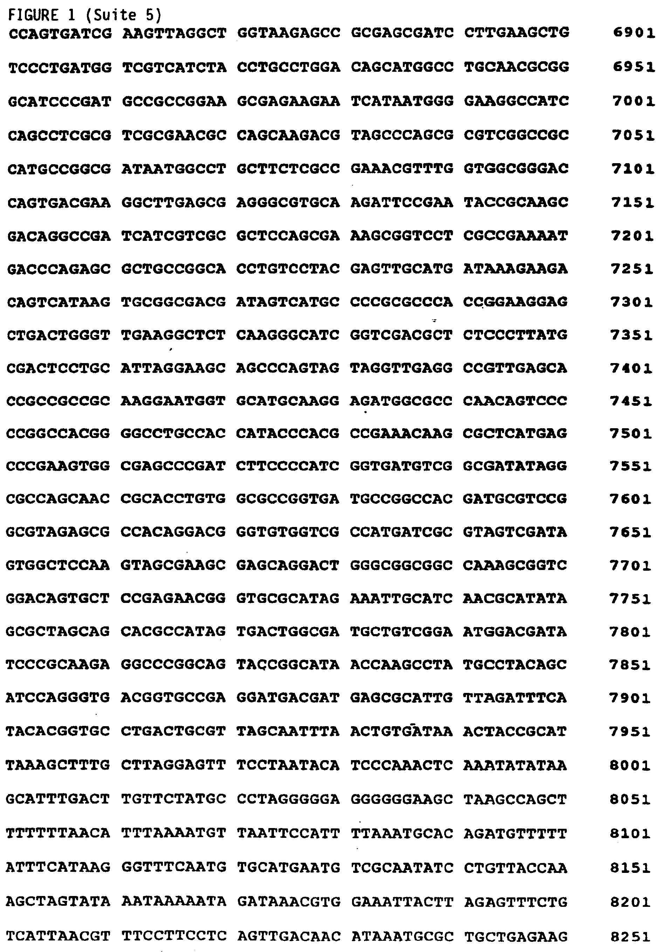

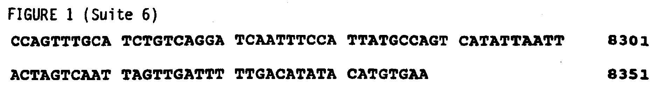

Selon une variante de réalisation de l'invention, la séquence provirale en amont du promoteur exogène est la séquence de nucléotides située entre les nucléotides 1 et 1500 environ selon la numérotation de la séquence représentée à la figure 1. Cette séquence en amont du promoteur est essentiellement dépourvue de l'ensemble des gènes gag, pol et env de la séquence provirale.

La séquence de nucléotides exogène est avantageusement insérée sous le contrôle du promoteur exogène, à la place des séquences gag, pol et env délétées.

Lorsque le promoteur est le promoteur PGK-1 décrit plus loin, la séquence de nucléotides exogène est avantageusement insérée au site BamHI en aval de ce promoteur.

### 2) Séquence de nucléotides exogène

Une "séquence de nucléotides exogène" selon l'invention est une séquence qui n'est pas exprimée naturellement dans une cellule dans laquelle on introduit le vecteur rétroviral de l'invention, ou y est exprimée de façon insuffisante, ou dont on veut obtenir une expression plus importante que l'expression normale. Ce défaut d'expression ou cette insuffisante d'expression résultent de la nature de la cellule ou survient à cause d'une pathologie affectant cette cellule chez un individu donné.

De façon préférée, la "séquence de nucléotides exogène" code pour un polypeptide déterminé. Par "polypeptide", on entend toute séquence d'acides aminés, quelle que soit sa taille, cette expression comprenant les protéines et les peptides. Le polypeptide peut être sous forme glycosylée ou non glycosylée.

### 3) Promoteur exogène

Le promoteur utilisé pour la construction de rétrovirus recombinants selon l'invention est avantageusement exogène par rapport à la séquence dont il contrôle l'expression en ce sens qu'il ne lui est pas naturellement associé. On peut aussi utiliser le promoteur du gène exogène transféré.

Le promoteur utilisé dans le vecteur de l'invention peut être de type constitutif ou inductible. Ce promoteur exogène contrôle l'expression de la séquence de nucléotides exogène. Le promoteur peut, le cas échéant, être accompagné d'une séquence nucléique régulatrice par exemple activatrice ("enhancer") qui servirait à réguler son activité.

Dans le cas de promoteurs inductibles, des promoteurs tels le promoteur Mx de souris, les promoteurs comprenant un opérateur tétracycline ou encore des promoteurs régulés par des hormones, en particulier des stéroïdes, peuvent être utilisés. En ce qui concerne les promoteurs dits "constitutifs", l'utilisation de promoteurs internes actifs dans les fibroblastes au repos, tels le promoteur PGK ou un autre promoteur de gène de ménage est préférée.

De façon particulièrement avantageuse pour la réalisation d'un vecteur rétroviral selon l'invention, le promoteur constitutif exogène est un promoteur sans TATA box et en particulier le promoteur du gène de la phosphoglycérate kinase (PGK-1). Ce promoteur est soit le promoteur de souris ou le promoteur humain tel que décrit par Adra et al (Gene 60, 1987, 65-74).

L'insertion du promoteur PGK-1 dans un vecteur rétroviral permet le transfert du gène dans des fibroblastes cutanés, par exemple, et l'expression stable à haut niveau et à très long terme de ce gène après que les fibroblastes aient été implantés in vivo chez un receveur, par exemple un mammmifère.

D'autres promoteurs constitutifs peuvent être utilisés à la place du promoteur PGK-1. De façon générale, on aura recours à un promoteur actif dans les cellules que l'on cherche à transformer avec le vecteur rétroviral et en particulier des promoteurs actifs dans des fibroblastes à l'état quiescent, par exemple des promoteurs précédant les gènes du cytosquelette. On peut également citer le promoteur de la β-actine. (Kort et al., 1983, Nucl. Acides Res. 11: 8287-8301) ou le promoteur du gène de la vimentine (Rettlez et Basenga (1987), Mol. Cell. Biol. 7: 1676-1685). Le promoteur utilisé peut être dépourvu de "TATA box".

### 4) Réalisations préférentielles du vecteur rétroviral

Un premier vecteur rétroviral particulier dans le cadre de l'invention est tel qu'une séquence de nucléotides exogène et un promoteur constitutif exogène, ainsi que la séquence de l'ADN proviral, sont portés par un plasmide. A titre d'exemple, ces séquences peuvent être portées par le plasmide pBR322 qui permet l'introduction de l'ADN dans les lignées cellulaires dans lesquelles on cherche à produire le vecteur.

Selon un autre mode de réalisation avantageux de l'invention, un vecteur répondant aux définitions précédentes est en outre caractérisé en ce que l'ADN proviral est issu du rétrovirus Mo-MuLV. D'autres rétrovirus de la famille des MuLV peuvent être utilisés et l'on citera par exemple les rétrovirus HaSV ou F-MuLV.

De préférence, les séquences des gènes pol et env de l'ADN proviral sont totalement délétées. Dans ce cas, la séquence du gène gag peut être également entièrement délétée ou au contraire être conservée en partie, dès lors que l'ADN du provirus ainsi constitué n'est plus susceptible de se répliquer.

Avantageusement, un vecteur rétroviral tel que défini précédemment est caractérisé en ce que la région U3 du fragment LTR3' est délétée au niveau du nucléotide 2797 de la figure 1. Cette délétion correspond à une délétion d'un fragment situé entre les nucléotides 7935 et 8113 selon la numérotation de la séquence publiée par Shinnick et al (Nature 293, 543-548, 1981). Ce fragment délété de la séquence U3 du LTR3' comporte la région activatrice du LTR.

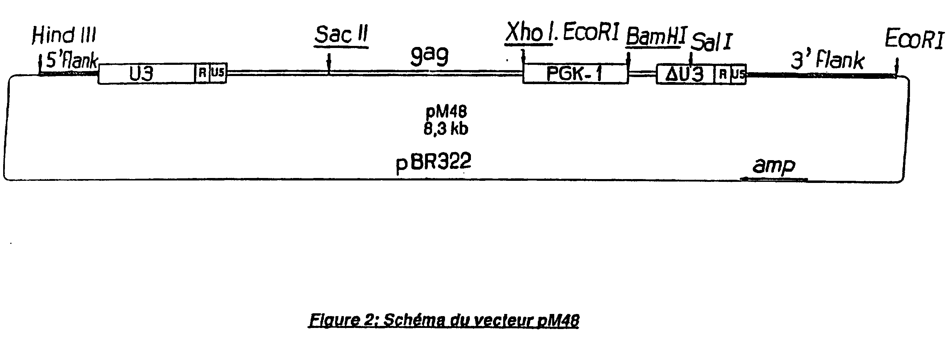

A titre préféré, un vecteur rétroviral selon l'invention est le vecteur de type pM48 dérivé du virus de Moloney dans lequel l'"enhancer" viral localisé en V3 a été supprimé et comportant un promoteur interne actif dans les fibroblastes au repos, de préférence le promoteur PGK ou un autre promoteur de gène de ménage, tel que le vecteur représenté à la figure 2, modifié par la séquence de nucléotides exogène au site BamHI.

Le vecteur rétroviral M48 LacZ (encore désigné par l'expression pM48 LacZ) a été déposé à la CNCM (Collection Nationale de Culture de Microorganismes, Paris - France) sous le N° I-1298, le 16 Avril 1993.

Ce vecteur est dérivé du vecteur pM48 et est caractérisé en ce qu'il contient en aval du promoteur constitutif exogène, un fragment BamHI du gène de la β-galactosidase.

Ce fragment du gène de la β-galactodidase peut aisément être délété et remplacé par une séquence de nucléotides exogène d'intérêt, par exemple une séquence codant pour une protéine ou une glycoprotéine susceptible d'avoir un intérêt thérapeutique ou contre le produit protéique de laquelle on souhaiterait par exemple obtenir des anticorps.

Un vecteur rétroviral de l'invention peut également contenir une séquence capable d'augmenter l'activité transcriptionnelle du promoteur, soit de manière constitutive, soit de manière inductible. Le vecteur peut contenir, en amont du promoteur exogène, une séquence d'activateur.

### b) Cellules recombinantes

L'invention concerne par ailleurs des cellules recombinantes, caractérisées en ce qu'il s'agit de cellules ayant la capacité d'être tolérées immunologiquement par un organisme chez lequel elles seraient implantées, modifiées par un vecteur rétroviral répondant à l'une des définitions précédentes.

De telles cellules, peuvent être des cellules issues de l' organisme chez lequel elles doivent être implantées après recombinaison par transduction avec le vecteur de l'invention, ou des cellules dépourvues à leur surface d'antigènes reconnus par le système immunitaire de l'organisme chez lequel elles sont implantées.

Il peut également s'agir de cellules endothéliales, de myoblastes, de cellules musculaires ou de cellules tumorales irradiées ou traitées selon d'autres procédés pour éviter leur prolifération et prélevées chez un patient et dont on souhaite modifier le patrimoine génétique afin qu'elles puissent intervenir sur le développement de la tumeur.

A titre préféré, ces cellules recombinantes sont des fibroblastes recombinants et en particulier des fibroblastes cutanés. De préférence, il s'agit de fibroblastes autologues par rapport au patient chez lequel on souhaite les implanter après leur modification par un vecteur selon l'invention. Des fibroblastes préparés à partir d'autres organes peuvent être utilisés, comme par exemple des fibroblastes isolés à partir d'un cordon ombilical. Les fibroblastes génétiquement modifiés et utilisés dans le contexte de la présente invention sont des fibroblastes non immortalisés.

Ces cellules peuvent être modifiées par un vecteur recombinant codant pour un polypeptide déterminé, ledit vecteur permettant l'expression de l'ADN étranger dans les cellules. Elles peuvent aussi être modifiées par les méthodes dites de recombinaison homologue. La pénétration du vecteur dans les cellules est réalisée en utilisant l'électroporation ou le précipité au phosphate de calcium ou encore par toute autre méthode impliquant l'entrée d'un acide nucléique soit seul soit par l'intermédiaire d'un virus recombinant par exemple.

Par "cellules recombinées", on entend également des cellules autologues tumorales irradiées préalablement à leur introduction dans le gel, ayant la capacité de maintenir à leur surface des antigènes tumoraux accessibles au système immunitaire de l'hôte chez lequel l'implant selon l'invention a été introduit.

Les cellules recombinantes de l'invention sont également obtenues soit par l'infection des cellules à modifier avec un vecteur rétroviral de l'invention, soit par d'autres méthodes de transduction ADN nu, complexe ADN-protéines ou vecteur adénovirus. Dans le cas d'une infection avec un vecteur rétroviral, la séquence codant pour le polypeptide dont on recherche l'expression (séquence de nucléotides .exogène) est introduite dans l'ADN proviral.

De manière préférée, les cellules recombinantes sont modifiées par un vecteur contenant une séquence ou plusieurs séquences de nucléotides exogène(s), codant pour un antigène ou un déterminant antigénique ou codant pour un polypeptide ou une glycoprotéine, soluble dans le sérum, par exemple pour un polypeptide ou une glycoprotéine d'intérêt thérapeutique, notamment pour une hormone, une protéine ou une glycoprotéine de structure ou une protéine ou une glycoprotéine du métabolisme ou une protéine ou une glycoprotéine virale ou une protéine ayant les caractéristiques d'un anticorps ou d'un fragment d'anticorps.

A titre d'exemple, cette séquence de nucléotides exogène code pour la β-glucuronidase, ou une autre enzyme lysosomale telle que l'α-iduronidase ou l'arylsulfatase B, un facteur de coagulation tel que le facteur VIII ou le facteur IX, l'érythropoïétine, ou toute partie active de l'une de ces protéines.

D'autres avantages et caractéristiques de l'invention apparaissent dans les exemples et les figures qui suivent.
- Figure 1 : séquence de nucléotides du vecteur M48 ;
- Figure 2 : représentation du vecteur PM48.

### EXEMPLES

### PREPARATION DU VECTEUR RECOMBINANT

Le transfert et l'expression de matériel génétique exogène permet de modifier expérimentalement les propriétés d'un tissu. Des traitements reposant sur ce principe pourraient être proposés à des malades présentant des déficiences génétiques ou des maladies acquises. L'exemple décrit ci-dessous fait appel à un vecteur rétroviral pour introduire dans des cellules l'information génétique codant pour une protéine secrétée. La réimplantation dans l'organisme des cellules génétiquement modifiées est réalisée en associant ces cellules avec du collagène, un facteur angiogénique et l'armature de type corail ou collagène réticulé. L'expression in vivo à long terme du matériel génétique ainsi transféré est obtenue en utilisant le promoteur du gène murin de la phosphoglycérate kinase.

### 1 - VECTEUR RETROVIRAL

### 1.1 - Caractéristiques

Le vecteur rétroviral M48 (figure 2) est construit à partir de séquences provirales isolées de l'ADN génomique de cellules de rat infectées par le rétrovirus leucémogène murin de Moloney (Mo-MuLV). Les gènes gag, pol et env qui codent pour les protéines du virus ont été retirés et remplacés par les séquences à véhiculer. On a conservé les séquences rétrovirales nécessaires en cis à la production et à la maturation des transcrits du génome viral recombinant, à leur empaquetage dans des particules virales infectieuses, à leur transcription inverse et à leur intégration dans le génome de la cellule infectée. Les séquences du provirus recombinant et les fragments flanquants d'ADN cellulaire sont portés par le plasmide bactérien pBR322.

Le promoteur du gène de la phosphoglycérate kinase (PGK-1) de souris a été inséré à la place des gènes viraux. Un site de clonage unique BamHI permet de placer les séquences à véhiculer dans le vecteur rétroviral sous le contrôle de ce promoteur.

Ce vecteur rétroviral comporte une délétion dans les séquences U3 du LTR interne (Cone et al., Mol. Cel. Biol. **7**, 887-897, 1987). Ceci entraîne, dans la cellule infectée, une forte diminution des ARNm démarrant au niveau du LTR 5', et une prédominance des ARNm produits à partir du promoteur PGK-1. L'utilisation de ce vecteur résulte donc en l'insertion, dans le génome de la cellule cible d'une cassette d'expression de la séquence d'intérêt sous le contrôle du promoteur PGK-1.

### 1.2. - Structure du vecteur

La séquence complète du vecteur M48 (Figure 1) comprend :
- **Position 1 à 2019 :** Séquences du Mo-MuLV comprenant la longue répétition terminale 5'(ou 5'LTR ou LTR5'), les séquences d'empaquetage Ψ+ qui incluent une partie du gène gag (position -447 à 1564 dans la numérotation de Shinnick et al. (Nature **293**, 543-548, 1981). Ces séquences sont modifiées de la façon suivante : insertion d'un linker Sac II (5'CCCGCGGG3') en position 1074 (position 626, Shinnick et al.).

Ceci conduit à une mutation en phase avec la séquence codant pour gag.
- **Position 2020 à 2526 :** Promoteur du gène de souris codant pour la phosphoglycérate kinase (position -524 à -20, avant le codon de début de traduction, d'après Adra et al. Gene 60, 65-74, 1987).
- **Position 2527 à 2532 :** Site unique BamHI pour l'insertion de séquences qui seront exprimées sous le contrôle du promoteur PGK-1.
- **Position 2533 à 3101 :** Séquences du Mo-MuLV comprenant l'extrémité 3' du gène env (32 codons) et le LTR3'. Ces séquences sont modifiées de la façon suivante : délétion des séquences U3 du LTR comprises entre 7935 et 8113 dans la numérotation de Shinnick et al. et insertion d'un linker Sal I (2798 dans la numérotation présente).
- **Position 3102 à 3674 :** Séquences génomiques de rat.
- **Position 3675 à 8003 :** Séquences du plasmide vecteur pBR322 (position 4363 à 29) comprenant le gène de β-lactamase. Ces séquences sont modifiées de la façon suivante : destruction du site BamH I (position 7657, numérotation présente).
- **Position 8004 à 8388 :** Séquences génomiques de rat.

### 1.3. - Production de vecteur rétroviral

Cette production s'effectue en introduisant la structure provirale recombinante dans une lignée cellulaire où les gènes gag, pol et env sont exprimés constitutivement.

Cette lignée, dite transcomplémentante ou d'empaquetage synthétise des particules rétrovirales dépourvues d'ARN génomique. La lignée ΨCRIP, dérivée de fibroblastes de souris NIH/3T3 est utilisée ici (Danos et Mulligan Proc. Natl. Acad. Sci (USA) **85**, 6460-6465, 1988). Lorsque la construction recombinante est introduite par transfection, les ARN transcrits à partir du LTR5' sont encapsidés et les cellules produisent alors des particules capables de transmettre le génome recombinant mais incapables de se répliquer.

Après transfection de la construction recombinante, les cellules productrices de vecteur rétroviral sont sélectionnées. On utilise pour la préparation de vecteur, soit un clone producteur de hauts titres viraux, soit une population polyclonale. Les étapes de l'isolement d'un clone producteur sont les suivantes :
1) Les cellules ΨCRIP sont transfectées par un coprécipité au phosphate de calcium (Graham et Van Der Erb, Virology, **52**, 456, 1973) de la construction rétrovirale et du plasmide pSV2neo (Southern and Berg, J. Mol. Appl. Gen. **1**, 327-341, 1982), dans un rapport molaire de 10 pour 1.
2) Les cellules ayant intégré de manière stable et exprimant l'ADN exogène sont sélectionnées en présence de G418 à 1 mg/ml (Geneticin, Gibco).
3) La population de cellules résistantes au G418 où des clones individuels isolés sont testés pour leur capacité à produire le vecteur rétroviral. Pour cela, des cellules cibles NIH/3T3 sont mises en présence du milieu de culture recueilli à partir de cellules ΨCRIP, et la transmission du provirus recombinant est analysée après 48 heures. L'analyse, effectuée à partir de l'ADN génomique des cellules cibles, se fait par PCR (méthode qualitative) ou par Southern blot (méthode quantitative).
4) Une banque de cellules productrices est conservée à - 135°C.

### 2 - PREPARATION DES STOCKS DE VECTEUR RETROVIRAL

Le surnageant de culture des cellules d'empaquetage est recueilli après une incubation de 24 heures sur les cellules d'empaquetage produisant le vecteur rétroviral, et mis en contact avec les cellules cibles pour réaliser l'infection.

### 2.1. - Préparation en masse

Un stock d'une centaine d'ampoules contenant des cellules productrices est conservé à -135°C. Après décongélation, les cellules sont amplifiées pendant 10 jours, successivement en flacon puis en rollers de 875 cm², jusqu'à obtention de 20 rollers confluents. La production est alors commencée. Elle dure 4 ou 5 jours. Le milieu de culture est du DMEM contenant 1 g/l de glucose, 10 mM de pyruvate de Na, et additionné avec 5 % de sérum de veau nouveau-né (Hyclone). Chaque roller permet le conditionnement de 100 ml de milieu par 24 heures. Ce surnageant de culture est centrifugé pour éliminer les débris cellulaires. La production hebdomadaire est de 8 à 10 litres de surnageant viral.

### 2.2. - Concentration

Le surnageant peut être concentré pour augmenter le titre infectieux du vecteur rétroviral. Après centrifugation, le surnageant est dialysé tangentiellement contre une membrane Sartocon de porosité 100.000, en utilisant le dispositif Crossflow de Sartorius. Une concentration de 20 fois est obtenue en 3 heures. Elle s'accompagne d'une augmentation de 20 fois des titres infectieux. Après concentration, la préparation virale est utilisée immédiatement, ou conservée à -80°C si le délai d'utilisation est supérieur à 2 semaines.

### 3 - FIBROBLASTES CUTANES

### 3.1. - Isolement et mise en culture

Les fibroblastes destinés au transfert de gène sont obtenus par une biopsie cutanée effectuée en conditions d'aseptie très soigneuse. Chez la souris, un animal syngénique est sacrifié à cet usage. Chez le gros mammifère, un ou plusieurs fragments de peau d'environ 10 cm² sont prélevés au cours d'une anesthésie générale. Après découpage en lambeaux de quelques mm², les fragments sont dissociés par un traitement enzymatique. Pour un fragment de 10cm², la réaction est effectuée pendant 2 heures à 37°C en agitation douce dans 50ml de milieu RPMI 1640 additionné de 10 % de sérum de veau foetal, 100 mg de collagénase (Worthington), 500 U de dispase (Collaborative Research Inc.). Après centrifugation et lavage, les cellules sont comptées, puis ensemencées dans des flacons de 150 cm² à la concentration de 1 à 5x10⁶/cm2. Le milieu de culture est du RPMI 1640 additionné de 10 à 20 % de sérum de veau foetal.

### 3.2. - Infection par le vecteur rétroviral

Les infections avec la préparation de vecteur rétroviral sont débutées le lendemain de la mise en culture, alors que la densité cellulaire est inférieure à 20 % de confluence. Elles sont répétées quotidiennement, suivant le même protocole pendant 4 ou 5 jours. Après retrait du milieu de culture, les cellules sont mises en contact avec la préparation de vecteur rétroviral non concentrée ou concentrée, dans tous les cas, additionnée avec 8µg/ml de polybrene. L'incubation est effectuée pendant 2 heures à 37°C, puis, sans lavage préalable, la préparation virale est remplacée par du milieu de culture.

Lorsqu'un vecteur codant pour une protéine détectable à l'examen microscopique est utilisé, on constate que ce procédé permet l'introduction du gène étranger dans plus de 90 % de fibroblastes. Le niveau de sécrétion de la protéine dans le surnageant de culture peut être contrôlé au cours et à la fin de ce processus. Les cellules peuvent être congelées en partie ou en totalité à ce stade.

Après infection, les cellules sont amplifiées jusqu'à obtention d'un nombre suffisant pour la réimplantation, soit 2-6x10⁸ cellules/kg de poids corporel. L'amplification est réalisée dans des plateaux de culture (multitray, Nunc).

### 3.3. - Recueil des cellules génétiquement modifiées.

Après amplification, les cellules sont recueillies par traitement à la trypsine. Après lavage et comptage, elles sont disponibles pour servir à la formation d'un neo-organe. Un échantillon est conservé pour contrôler le transfert génétique par Southern blot, ainsi que l'expression et la sécrétion de la protéine étrangère par une technique appropriée.

### Exemple 1 :

### Sécrétion d'une enzyme lysosomale (β-glucuronidase) chez la souris avec des implants contenant des fibres de PTFE, du collagène de queue de rat et des fibroblastes cutanés modifiés avec un vecteur rétroviral.

### 1.1. - Constituants

Trois composants sont assemblés in vitro. La méthode décrite ci-dessous est applicable à la réalisation de néo-organes de 1 ml contenant 5x10⁶ à 10⁷ cellules génétiquement modifiées. Les mêmes proportions sont utilisées pour la formation de néo-organes de grande taille contenant 1 à 5x10⁹ cellules.
1) Des fibres de polytetrafluoroéthylène (Gore et Associates) stérilisées par autoclavage (120°C, 30 minutes) sont recouvertes par du collagène de type 1. Les fibres sont baignées dans une solution de collagène de queue de rat (Sigma) à 0.1 % dans de l'acide acétique 0.1N. Ce traitement s'effectue sous vide pendant 1 heure à température ambiante, afin de chasser l'air présent entre les fibres synthétiques. Les collagènes d'origine murine, bovine ou humaine peuvent être indifféremment utilisés. Après séchage pendant 24 heures sous une hotte à flux luminaire, les fibres ainsi entourées d'une fine pellicule de collagène, sont incubées en présence d'un facteur de croissance angiogénique (FGF basique, Farmitalia, 10-20 ng/50 mg de fibres dans une solution de PBS-5% β-mercaptoethanol). Les fibres ainsi traitées, sont lavées en PBS et conservées à 4°C.
2) Une solution constituée des éléments suivants est préparée. Pour 1 ml : RPMI 1640 10x (Gibco), 100 µl ; bicarbonate 7,5 %, 12 µl ; NaOH 7,5 N, 8 µl ; Hepes 1 M, 2,5 µl ; Penicilline/Glutamine (Gibco), 100 µg ; bFGF (Farmitalia) 10 ng ; collagène (Bioethica) 1,5 mg ; eau distillée qsp 1 ml. Cette solution de consistance sirupeuse, est conservée à + 4°C.
3) Après lavage, les cellules génétiquement modifiées sont resuspendues dans 100 µl de RPMI 1640 avec 15 % de sérum de veau foetal.

### 1.2. - Assemblage

L'assemblage in vitro du néo-organe s'effectue stérilement sous hotte, de la manière suivante :
1) 1 mg de fibres traitées sont étalées dans une boîte de culture (plaque Corning 24 puits).
2) Les cellules génétiquement modifiées (100µl) sont incorporées à la solution de collagène (1ml), à l'aide d'un agitateur,
3) Ce mélange est déposé sur les fibres.

L'ensemble est placé dans un incubateur (37°C, 5 % CO_{z}) pendant 30 minutes afin d'obtenir la gélification du collagène. Lorsque celle-ci survient, lml de RPMI 1640 avec sérum de veau foetal est déposé sur le gel afin de compenser les variations de pH. Deux à quatre heures plus tard, le gel est délicatement décollé du bord de la boîte de culture à l'aide d'une aiguille à injection et transféré dans une boite plus large, en présence d'un excès de milieu de culture pour une période de 12 à 24 heures. Pendant ce laps de temps, les fibroblastes inclus dans le gel de collagène s'attachent sur les fibres de collagène, ce qui induit une rétraction de la structure. Il est habituel de constater une réduction de plus de 50 % du volume initial. L'ensemble est alors prêt à être implanté dans l'organisme.

### 1.3. - Implantation

Le néo-organe est introduit dans la cavité péritonéale au cours d'une laparotomie effectuée sous anesthésie générale. Chez la souris, l'implantation est effectuée en insérant sans fixation le néo-organe entre les anses intestinales au contact du mésentère. Chez le gros mammifère, le néo-organe est fixé par deux points entre les deux feuillets du tablier épiploïque. Des connexions vasculaires s'établissent dès les premiers jours. La protéine exogène peut alors être libérée dans la circulation de l'animal receveur. A partir de la troisième semaine suivant l'implantation, un organe souvent pédiculé, encapsulé et bien individualisé est formé. De nombreuses connexions vasculaires sont visibles à sa surface. Il n'existe pas d'adhérence inflammatoire. Les examens après 3 et 6 mois montrent un aspect identique. L'analyse histologique à ce stade révèle la présence d'un tissu conjonctif lâche, vascularisé et peu inflammatoire.

### 1.4. - Traitement des maladies de surcharge lysosomale.

Ce procédé a été appliqué au traitement d'une maladie de surcharge lysosomale pour laquelle un modèle murin est disponible. Il s'agit d'un déficit en β-glucuronidase responsable d'une mucopolysaccharidose. La β-glucuronidase est phosphorylée et est donc, en partie, transportée vers les lysosomes et en partie, secrétée dans la circulation. Elle peut être captée par d'autres tissus, par l'intermédiaire du récepteur au mannose-6-phosphate exprimé à la surface de nombreuses cellules. Ce captage spécifique permet une approche thérapeutique où la distribution systémique continue de l'enzyme manquante est assurée par des cellules génétiquement modifiées.

Des implants de 0,5 cm³ contenant 20x10⁶ fibroblastes autologues sont bien tolérés par 1a souris. Les cellules implantées expriment le gène de la β-glucuronidase humaine inséré dans le vecteur M48, sous le contrôle du promoteur PGK et sécrètent la protéine étrangère au-delà de 5 mois. L'effet biologique est objectivé par une chute rapide de l'élimination urinaire des catabolites intermédiaires de la dégradation des mucopolysaccharides. La normalisation histologique des tissus de l'animal receveur est spectaculaire, particulièrement au niveau du foie et de la rate. Elle est stable au cours du temps, et dépend de la présence du néo-organe dont l'exérèse induit un retour à l'état pathologique initial.

Ce procédé est applicable au traitement de l'ensemble des maladies de surcharge lysosomale humaines, y compris la maladie de Gaucher pour laquelle l'apport d'une forme soluble de la glucocérébrosidase est envisageable.

### Exemple 2 :

### Secrétion d'une enzyme lysosomale (β-glucuronidase) chez le chien avec des implants contenant des fibres de PTFE, du collagène de queue de rat et des fibroblastes cutanés modifiés avec un vecteur rétroviral.

Quatre chiens ont reçu de 1 à 6 néo-organes contenant chacun 10⁹ fibroblastes autologues génétiquement modifiés au moyen du vecteur M48-βglu, pour secréter la β-glucuronidase humaine. Trois animaux de race Labrador pesant de 21 à 29 kg, et un animal de race Beagle pesant environ 9 kg ont été utilisés. L'implantation chirurgicale réalisée sous anesthésie générale a consisté à insérer les implants entre les deux feuillets pariétal et viscéral du tablier épiploique à proximité de la grande courbure de l'estomac. Ce geste rapide s'acccompagne de suites opératoires simples, et permet d'envisager pour l'avenir une implantation par coeliochirurgie. Un mois et demi, quatre mois, et six mois après l'implantation, une laparotomie exploratrice a été réalisée afin de pratiquer un bilan macroscopique de l'implant. La mise à jour du tablier épiploïque a été facile et le repérage de la structure greffée s'est fait très rapidement. Aucune adhérence n'a été notée, et la vascularisation était importante, comportant des vaisseaux de gros calibre en connexion avec 1 'implant. L'aspect de l'implant ne s'est pas modifié pendant la durée de cette observation. Une biopsie hépatique pratiquée dans le même temps opératoire a permis de rechercher la présence de β-glucuronidase humaine. La mesure de l'activité sur des extraits cellulaires a montré un niveau équivalent à 2-3% de l'activité canine endogène, stable au cours du temps chez les animaux ayant reçu plus de 2 x 10⁹ fibroblastes autologues génétiquement modifiés. La révélation in situ sur coupe a montré une activité localisée dans les cellules de Küpffer. Les résultats sont résumés au tableau I.

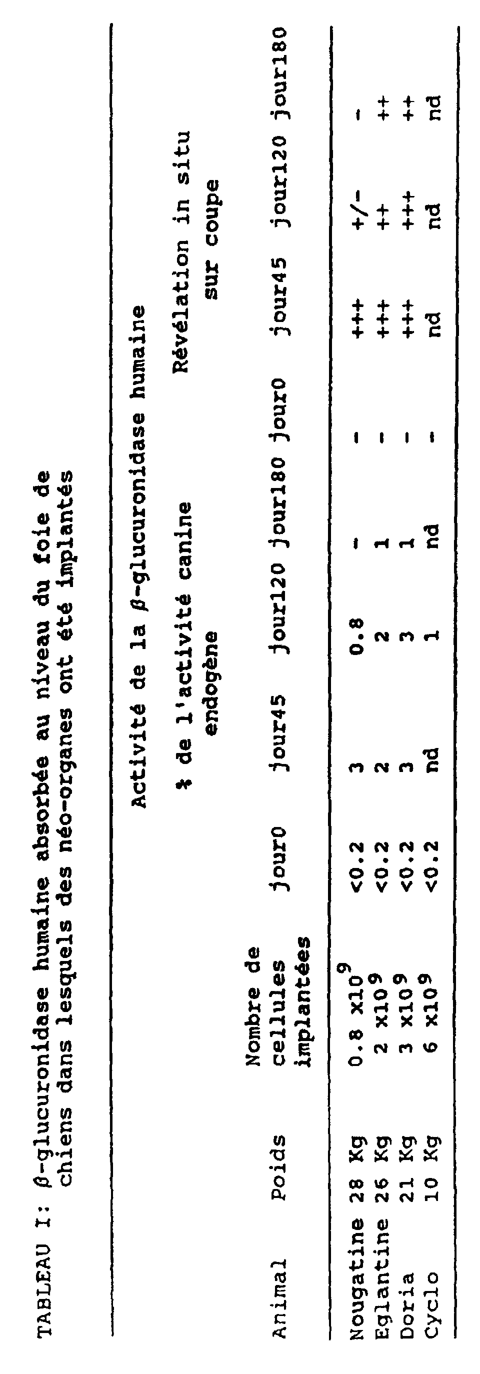

### Exemple 3 :

### Secrétion d'une enzyme lysosomale (β-L-iduronidase) chez la souris avec des implants contenant des fibres de PTFE, du collagène de queue de rat et des fibroblastes cutanés modifiés avec un vecteur rétroviral.

Un cDNA codant pour l'β-L-iduronidase humaine a été introduit dans le vecteur M48 et un rétrovirus recombinant a été produit dans la lignée ΨCRIP. Des fibroblastes de souris nude ont été placés en culture primaire et infectés par ce vecteur rétroviral. Les cellules secrétant l'enzyme humaine ont été introduites chez six receveurs syngéniques, dans deux néo-organes contenant chacun 10 millions de cellules.

Les animaux ont été sacrifiés après 35 à 77 jours et la présence d'enzyme humaine dans leur foie et leur rate a été vérifiée au moyen d'un anticorps monoclonal. Une activité enzymatique équivalente à 1-2% de l'activité endogène a été mise en évidence, indiquant la production de l'enzyme à partir des cellules du néo-organe et son captage à distance.

### Exemple 4 :

### Secrétion d'érythropoiétine (EPO) chez la souris avec des implants contenant des fibres de PTFE, du collagène de queue de rat et des fibroblastes cutanés modifiés avec un vecteur rétroviral;

Le gène nlslacZ a été excisé du vecteur M48-nlslacZ par digestion BamHI et remplacé par un cDNA codant pour l'EPO de souris synthétisé par PCR, réalisant ainsi le vecteur M48EPO. Un clone de cellule ΨCRE produisant des particules rétrovirales M48EPO a été isolé. Des fibroblastes ont été prélevés par biopsie cutanée chez des souris adultes et une culture primaire a été établie en milieu RPMI 1640 avec 10% de SVF. Ces cellules ont été infectées par le fecteur M48EPO de manière itérative durant les 4 premiers jours de la culture. L'analyse par Southern blot amontré que les cellules infectées contenaient en moyenne 2 copies du génome M48 EPO par cellule. L'analyse par Northern blot a montré une expression du cDNA de l'EPO très majoritairement à partir du promoteur PGK-1. Un essai biologique de l'activité EPO sécrétée par ces cellules, ainsi qu'un test ELISA ont mesuré une secrétion de 17 unités d'EPO par million de cellules par 24 heures. Ces cellules ont été amplifiées en culture, trypsinées, puis resuspendues dans du RPMI 1640 à une concentration de 2,5 x 10⁷ à 2 x 10⁸ cellules par ml.

Des implants ont été préparés en associant les éléments suivants dans un puits de 0,9 cm²/ 1) des fibres de PTFE préalablement traitées comme décrit ci-dessus pour être recouvertes successivement de collagène de queue de rat (sigma), d'héparine (Roche), et de bFGF (Promega) ; 2) 1 ml d'une solution contenant du collagène de queue de rat (Sigma) à x mg/ml, du bFGF (10µg/ml) dans du milieu RPMI 1640 ; 3) un volume de 10µl de la suspension de fibroblastes génétiquement modifiés pour secréter de l'EPO. Après avoir réalisé un mélange homogène, celui-ci est solidifié par incubation à 37°C pendant 30 minutes, puis le gel est délicatement séparé des parois du puits de culture, transféré dans une boîte de Petri de 35 mm de diamètre, recouvert de milieu RPMI 1640 contenant 10% de SVF, et incubé à 37°C pendant 3 jours. Une contraction du gel s'est opérée durant cette incubation résultant en une diminution de taille de 50%. Les implants ont ensuite été insérés dans la cavité péritonéale de 14 souris syngénéiques aux fibroblastes génétiquement modifiés.

La mesure hebdomadaire de l'hématocrite (valeur normale 46 ± 1,5%) a montré une élévation progressive vers un plateau dont le niveau variait en fonction du nombre de cellules secrétant de l'EPO dans les implants. Ce plateau était à 80% pour 2 x 10⁷ cellules, 70% pour 10⁷ cellules, 60% pour 5 x 10⁶ cellules, 52% pour 2,5 x 10⁶ cellules. Il s'est maintenu à ce niveau durant les 6 mois d'observation des animaux. Les concentrations d'EPO dans le sérum des souris porteuses d'implants secrétant de l'EPO étaient de 60 à 400 mU par ml (normale < 20 mU/ml).

Les implants constitués de PTFE, de collagène de queue de rat et de cellules génétiquement modifiées par le vecteur rétroviral M48EPO permettent donc une secrétion d'EPO in vivo stable à long terme, à des niveaux élevés estimés entre 500 et 1500 mU/kg/24 heures, c'est-à-dire suffisants chez l'homme pour la correction de l'anémie due aux hémoglobinopathies comme le drépanocytose et la β-thalassémie. DEs niveaux 10 fois moindres seraient suffisants pour le traitement de l'anémie associée à l'insuffisance rénale chronique.

### UTILISATION IN VIVO D'IMPLANTS COMPRENANT DU CORAIL A TITRE DE SUPPORT BIOLOGIQUE

### 1. Chez la souris

Chez la souris C3H/He des expériences de compatibilité du Biocoral® en tant qu'implant, ont montré qu'il pouvait représenter une alternative avantageuse par rapport à des supports synthétiques.

Le Biocoral® a pu en effet être efficacement recouvert de différents éléments de la matrice extra cellulaire ainsi que de facteurs angiogéniques. On a montré que le collagène de type I murin à une concentration de 0.5 mg/ml dans 0.001% d'acide acétique adhère et recouvre le support corallien après séchage à l'air ambiant pendant 12 à 24 hrs. La présence de cet élément de la matrice extra-cellulaire a ensuite permis la liaison irréversible de facteur basique de croissance des fibroblastes marqué avec ¹²⁵I ¹²⁵I-basic Fibroblast Growth Factor (bFGF) au Biocoral®. On a réalisé ainsi la fixation en moyenne de 50 ng de bFGF sur 40 mg de support corallien prétraité au collagène murin de type I. Bien que cette liaison ait pu s'effectuer aussi en l'absence de prétraitement au collagène, elle était dans ce cas réversible donc de faible affinité. Le collagène est donc un intermédiaire indispensable et justifié pour la fixation stable de facteurs angiogéniques au Biocoral. Enfin, la liaison du bFGF a pu être augmentée de cinq à dix fois lorsque le recouvrement de 40 mg de Biocoral par du collagène était complété par de l'héparan sulfate (Sigma) 200 µg (solution à 1 mg/ml dans du PBS) ou de l'héparine (Roche) 500 unités (solution à 5000 unités/ml). Ces deux composants de la matrice extra-cellulaire ont une affinité connue pour le collagène (Baird, A., Schubert, D., Ling, N., and Guillemin, R. (1988) Receptor and heparine binding domains of basic fibroblast growth factor. Proc. Natl. Acad. Sci. USA 85, 2324-2328). Leurs caractères anionique et poly-sulfaté sont responsables de la forte affinité pour le bFGF (Johnson, D.E., Lee, P.L., Lu, J., and Williams, L.T. (1990) Diverse forms of a receptor for acidic and basic fibroblast growth factors. Mol. Cell. Biol. 10, 4728-4739). Après incubation en présence de l'un ou l'autre de ces composants, 1 hr à 4°C sous agitation, le Biocorail a été séché à l'air ambiant. Ainsi héparine, le corail a une action anticoagulante.

Des essais d'implantation sous-cutanée de corail sphérique de 40 mg, à haute porosité (50%), recouvert de collagène de type I murin et de bFGF (=50 ng/40 mg de corail) ont été réalisés in vivo sur une dizaine de souris. L'aspect macroscopique et histologique de ces implants a été analysé sur une période de 7 mois. Les résultats montrent que le Biocoral était progressivement résorbé en quelques mois et laissait place à une structure formée de tissu conjonctif vascularisé ayant la forme sphérique initiale de l'implant corallien avec cependant une réduction de volume d'approximativement un tiers. L'analyse histologique de ce tissu conjonctif à trois mois, montrait une vascularisation intense et l'absence totale de cellules inflammatoires en regard des résidus coralliens. Le tissu conjonctif proprement dit était formé de cellules mésenchymateuses peu denses, entourées de fibres de collagène néo-formé. L'analyse à 7 mois montrait des résultats semblables sans involution.

### 2. Chez le chien :

Chez un chien de 25 kg, nous avons réalisé une première expérience d'implantation de néo-organe dont la partie externe en corail poreux (50%) a permis de retenir un gel de collagène rétracté contenant 1 à 2 x 10⁹ fibroblastes autologues génétiquement modifiés. Le support en Biocoral avait été recouvert par du collagène murin dé type I et de l'héparine (Roche). Après séchage, le corail ainsi prétraité a été incubé en présence de facteur de croissance angiogénique de type bFGF (Farmitalia) puis rincé en PBS. L'implantation chirurgicale réalisée sous anesthésie générale, a consisté à insérer l'ensemble entre les deux feuillets pariétal et viscéral du tablier épiploïque à proximité de la grande courbure de l'estomac. Ce geste rapide a permis des suites opératoires simples, et laisse envisager pour l'avenir une implantation par coeliochirurgie. Quarante cinq jours après, une laparotomie exploratrice a été réalisée afin de pratiquer un bilan macroscopique de l'implant. La mise à jour du tablier épiploïque a été facile, sans adhérence et le repérage de l'implant corallien, contenant les fibroblastes autologues génétiquement modifiés, s'est fait très rapidement. Aucune adhérence n'a pu être notée, et la vascularisation était importante, comportant des vaisseaux de gros calibre en connexion avec l'implant. Un processus de résorption progressive du corail a été observé pendant 4 mois au moins.

### 3. Autres mammifères :

La création de néo-organes, en utilisant du Biocoral, adaptés à l'implantation chirurgicale sur de gros mammifères, notamment chez l'homme, en vue d'un développement clinique est réalisée de façon analogue. Le Biocoral est modulable dans sa conception, capable de déclencher une angiogénèse soutenue associée à sa propre résorption et enfin dénué d'effets systémiques et locaux de type inflammatoire. Un tel bio-matériau sert avantageusement de receptacle et de squelette à un gel de collagène de type I contenant des cellules autologues génétiquement modifiées, capables de sécréter un facteur thérapeutique dans la circulation une fois les connexions vasculaires établies.

### Exemple 5 :

### Sécrétion d'érythropoiétine (EPO) chez la souris avec des implants contenant des fragments de corail, du collagène de queue de rat et des fibroblastes cutanés modifiés avec un vecteur rétroviral.

De la même façon que dans l'exemple 4, des fibroblastes de souris ont été obtenus à partir d'une biopsie cutanée, mis en culture, et transduits avec le vecteur M48EPO. Après amplification, les cellules ont été trypsinées et resuspendues dans du milieu RPMI 1640 avec 10% de SVFR à la concentration de 10⁸ cellules par ml.

Des implants ont été préparés en associant les éléments suivants dans un puits de 0,9 cm² : 1) de la poudre de corail (Inoteb) formée de particules de Porosité ≥ 45% et de granulométrie entre 600 et 1000 µm dont les pores avaient un diamètre moyen de 150 µm préalablement traitées par une incubation dans une solution de collagène de queue de rat (1 mg/ml) pendant 30 minutes à température ambiante, séchées, puis incubées dans une solution d'héparine (Roche) pendant 30 minutes à température ambiante, séchées, puis incubées dans une solution de bFGF (Promega) à 100 µg/ml pendant 30 minutes à température ambiante ; 2) 1 ml d'une solution contenant du collagène de queue de rat (Sigma) à 3 mg/ml, du bFGF (100 µg/ml) dans du milieu RPMI 1640 ; 3) un volume de 100 µl de la suspension de fibroblastes génétiquement modifiés pour secréter de l'EPO. Comme dans les exemples 1 à 3, un mélange homogène de ces constituants a été solidifé par incubation à 37°C pendant 30 minutes, puis le gel a été délicatement séparé des parois du puits de culture, transféré dans une boîte de Petri de 35 mm de diamètre, recouvert de milieu RPMI 1640 contenant 10% de SVF, et incubé à 37°C pendant 3 jours. Les implants ont ensuite été insérés dans la cavité péritonéale de 3 souris syngénéiques aux fibroblastes génétiquement modifiés.

Comme dans l'exemple 4, la mesure de l'hématocrite des animaux receveurs a montré une augmentation rapide atteignant un plateau après 3 semaines, et se maintenant à ce niveau pendant toute la durée de l'observation (2 mois). Des implants formés d'un gel de collagène structuré avec des fragments de corail permettent donc le maintien à l'état fonctionnel de fibroblastes génétiquement modifiés implantés dans la cavité péritonéale d'animaux receveurs, ainsi que la secrétion à long terme d'une protéine soluble dans le sérum.

### Exemple 6 :

### Sécrétion d'érythropoiétine (EPO) chez la souris avec des implants contenant des fibres de collagène réticulé, du collagène de queue de rat et des fibroblastes cutanés modifiés avec un vecteur rétroviral.

Une expérience semblable aux exemples 4 et 5 a consisté à réaliser des implants associant des fibroblastes de souris transduits avec le vecteur M48EPO, du collagène de queue de rat, et un support formé de fibres de collagène réticulé (Imedex). La préparation a été réalisée de la façon suivante : 1) les fibres déshydratées et stérilisées par rayonnement ont d'abord été réhydratées par une incubation de 12 heures à +4°C dans du milieu RPMI 1640. Elles ont ensuite été incubées à température ambiante pendant 30 minutes dans du milieu RPMI contenant 10% de SVF et 100 µg/ml de bFGF (Promega), puis disposées dans un puits de 0,4 cm² dans lequel a été ajouté un ml d'une solution de RPMI 1640 contenant du SVF (10%), du collagène de queue de rat (1 mg/ml), du bFGF (100 µg/ml, Promega), et 10⁷ fibroblastes génétiquement modifiés pour secréter de l'EPO. Après solidification à 37°C pendant 30 minutes, le gel a été délicatement décollé des parois du puits de culture, transféré dans une boîte de Petri de 35 mm de diamètre, et recouvert de milieu RPMI 1640 avec 10% de SVF. Les implants ont été incubés 3 jours à 37°C, une contraction modérée de la structure a été observée, puis ils ont été implantés chez 3 souris syngénéiques.

De manière similaire à ce qui a été décrit dans les exemples 4 et 5, la mesure hebdomadaire de l'hématrocrite a montré une élévation progressive jusqu'à un niveau plateau qui s'est maintenu pendant la durée de l'observation (2 mois). Des implants formés d'un gel de collagène structuré avec des fibres de collagène réticulé permettent donc le maintien à l'état fonctionnel de fibroblastes génétiquement modifiés implantés dans la cavité péritonéale d'animaux receveurs, ainsi que la secrétion à long terme d'une protéine soluble dans le sérum.

### Exemple 7 :

### Détoxification de la bilirubine par glucurono-conjugaison chez des rats porteurs d'implants contenant des fibres de PTFE, du collagène de queue de rat, et des fibroblastes génétiquement modifiés exprimant l'enzyme UDP-glucuronosyl transférase.

La bilirubine est un produit de dégradation de l'hème produit de manière permanente et en quantité importante par l'organisme. La bilirubine est liposoluble, et peut pénétrer passivement dans les cellules, où son accumulation est toxique. L'élimination de la bilirubine est réalisée par glucurono-conjugaison au niveau des hépatocytes par l'enzyme bilirubine UDP-glucuronosyl transférase (bil. UDPGT) dont il existe deux isoformes connues (1 et 2). L'addition d'acide glucuronique rend la molécule hydrosoluble, non toxique, et permet son excrétion dans la bile. Le déficit héréditaire total de l'activité bil.UDPGT est responsable d'une maladie gravissime (maladie de Crigler-Najjar) qui est redevable d'une transplantation hépatique. Il existe un modèle de cette maladie chez le rat (rat Gunn).

Le cDNA de rat codant pour l'isoforme 2 (bil.UDPGT-2) et le cDNA humain codant pour l'isoforme 1 (bil.UDPGT-1) ont été insérés dans le vecteur M48 après excision du gène nlslacZ par l'enzyme BamHI, générant ainsi les vecteurs M48GT-2 et M48GT-1. De manière semblable à ce qui a été décrit dans les exemples 1, 3 et 4 des fibroblastes de rat Gunn ont été prélevés par une biopsie cutanée, mis en culture, et transduits par le vecteur M48GT-2. La présence d'en moyenne une copie du génome vecteur dans les cellules transduites a été vérifiée par Southern blot, et l'expression à partir du promoteur PGK a été vérifiée par Northern Blot. L'activité enzymatique bil.UDPGT a été détectée dans les extraits microsomaux des fibroblastes infectés. Les cellules ont été amplifiées en culture, trypsinées, puis resuspendues dans du RPMI 1640 à une concentration de 2 x 10⁸ cellules par ml.

Des implants contenant 2 x 10⁷ cellules génétiquement modifiées ont été préparés de la même façon que dans les exemples 1, 3 et 4. Deux implants ont été insérés dans la cavité péritonéale de chaque rat Gunn receveur. Comme la colonie de rats Gunn utilisée n'est pas consanguine, il a été pris soin que chaque animal soit réimplanté avec ses propres cellules. La mesure des concentrations de bilirubine non conjuguée chez les animaux traités et chez des rats témoins du même âge a fait apparaître une diminution significative (entre 20 et 50%) et stable pendant une observation de 2 mois chez les rats porteurs d'implants de fibroblastes exprimant la bil.UDPGT. Des échantillons de bile ont été recueillis au moment du sacrifice des animaux et analysés par chromatographie liquide à haute pression (HPLC). Alors que les animaux non traités montrent une totale absence de bilirubine conjuguée dans la bile, les rats Gunn porteurs d'implants contenant des fibroblastes exprimant la bil.UDPGT présentaient des pics identifiés comme de la bilirubine mono et diconjuguée. La proportion de bilirubine conjuguée atteignait jusqu'à 11% de la bilirubine présente dans la bile.

Ces résultats indiquent que des fibroblastes de rat transduits avec le vecteur M48UDPGT-1 et greffés chez des rats Gunn sous la forme d'implants associant des fibres de PTFE et du collagène de queue de rat, sont capables de réaliser in vivo la réaction de glucurono-conjugaison de la bilirubine, laquelle bilirubine conjuguée peut ensuite être éliminée dans la bile par les hépatocytes, permettant ainsi une correction partielle du phénotype des animaux malades. Il est donc possible de réaliser la détoxification de produits biologiques in vivo grâce à des fibroblastes génétiquement modifiés implantés dans un gel de collagène structuré avec des fibres de PTFE.

### Exemple 8 :

### Utilisation d'un promoteur inductible (Mx) pour moduler l'expression d'un cDNA inséré dans un vecteur rétroviral.

Dans le cas d'implants secrétant de manière continue une protéine dans la circulation, il serait souhaitable de pouvoir exercer un contrôle sur cette secrétion. Pour ce faire un vecteur rétroviral dérivé du vecteur M48 a été construit dans lequel le promoteur PGK-1 a été excisé et remplacé par un fragment de 250 bp couvrant la région -250 / +1 du promoteur du gène Mx de la souris. Ce promoteur comprend des éléments de réponse à la stimulation par les interférons α et β. Chez la souris la protéine Mx est exprimée en réponse à une secrétion d'interféron α ou β. De la même façon que dans les exemples 3, 4 et 5, le cDNA codant pour l'EPO de souris a été inséré dans ce vecteur au site BamHI, générant ainsi le vecteur MxEPO. Des cellules ΨCRE produisant des particules rétrovirables écotropes recombinantes contenant le génome du vecteur MxEPO ont été isolées. Des fibroblastes isolés d'une biopsie cutanée réalisée chez une souris ont été infectés avec le vecteur MxEPO, et l'EPO secrétée dans le surnageant de ces cultures a été mesurée avant et après addition d'interféron α (1000 unités par ml) dans le milieu de culture. Les niveaux de base de secrétion d'EPO étaient inférieurs à 0,3 unités/ml/24 heures témoignant d'une faible activité du promoteur Mx présent dans le vecteur MxEPO en l'absence d'interféron. Une augmentation de la secrétion jusqu'à 10 fois le niveau de base a été mesurée après addition d'interféron α. Cette secrétion élevée s'est maintenue pendant au moins 6 jours. Cette observation montre qu'un contrôle peut être exercé sur un promoteur transcriptionnel inséré dans un vecteur rétroviral, après l'intégration de celui-ci dans le génome de la cellule cible.

Des implants constitués de fibres de PTFE, de collagène de queue de rat, et contenant des fibroblastes cutanés transduits avec le vecteur MxEPO ont été préparés et implantés chez des souris syngénéiques. En l'absence d'autre traitement, ces animaux ont maintenu un hématocrite normal. Certains des animaux receveurs ont été traités par l'injection intra-péritonéale de 50 mg de polyA-U (Boehringer) chaque 48 heures pendant deux semaines afin de provoquer une élévation de l'hématocrite.

### Traitement de maladies génétiques ou acquises.

Le procédé est applicable à la distribution dans le sérum de toute protéine soluble, ou de protéines transmembranaires tronquées de leur domaine d'immobilisation dans la membrane. Cependant, il ne permet pas une régulation des concentrations sériques de la protéine étrangère. Les applications suivantes sont envisagées pour les maladies génétiques : dans l'hémophilie B, l'apport de facteur IX ; dans l'hémophilie A, l'apport de facteur VIII sous forme d'une protéine délétée dans la région B ; dans la β-thalassémie, l'apport d'érythropoiétine susceptible de corriger l'anémie par stimulation de la synthèse d'hémoglobine foetale.

L'apport de CD4 soluble, ou de ses dérivés couplés à des immunotoxines ou des immunoglobulines, est envisageable dans les infections par des rétrovirus de la famille du VIH. Plus généralement, toute protéine anti-virale soluble pourrait être distribuée de cette façon.

### Production d'anticorps

La distribution de protéine étrangère chez la souris induit une réponse immunitaire marquée par l'apparition d'anticorps spécifiques, comme nous l'avons observé pour la β-glucuronidase, le CD4 soluble, et l'érythropoïétine. Le procédé pourrait donc être appliqué pour immuniser des animaux contre le produit non encore identifié d'un cDNA isolé par génétique inverse ou par d'autres moyens. Des anticorps polyclonaux et monoclonaux pourraient être obtenus de cette manière, sans nécessiter la préparation préalable de la protéine.

La même approche est envisageable pour réaliser des immunisations dans un but thérapeutique ou préventif. Des applications vaccinales pourraient être proposées, par exemple, contre des épitopes neutralisants de l'enveloppe du VIH ou des virus apparentés.

### Régulation par le système tel opérateur/tet répresseur

On prépare des fibroblastes cutanés dans lesquels on introduit deux vecteurs rétroviraux, l'un codant pour la protéine d'intérêt placé sous le contrôle d'un promoteur comportant les éléments tet opérateur, l'autre codant pour le tet répresseur ou une molécule apparentée modifiée pour produire un effet activateur. Suivant la nature de ce dernier élément et celle du promoteur contenant les séquences opératrices, l'addition de tétracycline induira une activation ou une répression de la transcription. Les fibroblastes transduits avec ces deux vecteurs sont implantés in vivo dans un néo-organe, et les animaux sont traités par de la tétracycline pour évaluer l'induction de l'expression. Le gène rapporteur est de préférence celui de l'EPO de souris.

### Régulation par le récepteur de la progestérone modifé pour induire la transcription en présence de RU486.

Le récepteur de la progestérone lorsqu'il est tronqué de sa région C-terminale devient capable de stimuler la transcription en présence de RU486. Des fibroblastes cutanés sont infectés avec deux vecteur rétroviraux, l'un codant pour la protéine d'intérêt exprimé sous contrôle d'un promoteur comportant des éléments de réponse à la progestérone, l'autre codant pour une version tronquée du récepteur de la progestérone. Comme précédemment, les fibroblastes transduits avec ces deux vecteurs sont implantés dans des néo-organes chez la souris, et les animaux sont traités par le RU486.

## Revendications

1. Implant **caractérisé en ce qu'**il comprend ;
- un support biocompatible permettant l'ancrage biologique de cellules ; et,
- des cellules eucaryotes ancrées dans et/ou à la surface dudit support biocompatible,
lesdites cellules eucaryotes ayant la capacité d'être tolérées immunologiquement par l'organisme dans lequel elles seraient susceptibles d'être implantées.

2. Implant selon la revendication 1, **caractérisé en ce que** le support biocompatible est un support à base de calcaire.

3. Implant selon la revendication 2, **caractérisé en ce que** le support biocompatible est à base de corail, de préférence de corail à haute porosité.

4. Implant selon l'une quelconque des revendications 1 à 3 **caractérisé en ce qu'**il comprend en plus un constituant capable d'induire et/ou de favoriser la gélification desdites cellules.

5. Implant selon l'une quelconque des revendications 1 à 4, **caractérisé en ce qu'**il comprend en plus un facteur angiogénique.

6. Implant selon l'une quelconque des revendications 1 à 5, **caractérisé en ce que** lesdites cellules eucaryotes sont impliquées dans le développement d'une structure comportant un tissu conjonctif vascularisé.

7. Implant selon l'une quelconque des revendications 1 à 6, **caractérisé en ce que** l'implant produit *in vivo* un effet thérapeutique.

8. Implant selon la revendication 7, **caractérisé en ce que** l'effet thérapeutique est le développement d'une structure comportant un tissu conjonctif vascularisé.

9. Implant selon la revendication 8, **caractérisé en ce que** le tissu conjonctif est au moins partiellement formé de cellules mésenchymateuses.

10. Implant selon l'une quelconque des revendications 1 à 9, **caractérisé en ce que** les cellules eucaryotes sont génétiquement modifiées.

11. Procédé de préparation d'un implant selon l'un quelconque des revendications 1 à 10, ledit procédé comprenant
- l'ancrage de cellules eucaryotes dans et/ou à la surface d'un support biocompatible, lesdites cellules ayant la capacité d'être tolérées immunologiquement par un hôte mammifère ; et,
- la récupération de l'implant ainsi obtenu.

12. Procédé selon la revendication 11, **caractérisé en ce que** le support biocompatible est un support à base de calcaire.

13. Procédé selon la revendication 12, **caractérisé en ce que** le support biocompatible est à base de corail, de préférence de corail à haute porosité.

14. Procédé selon l'une quelconque des revendications 11 à 13, **caractérisé en ce que** les cellules sont mises en contact avec un constituant capable d'induire et/ou de favoriser leur gélification.

15. Procédé selon l'une quelconque des revendications 11 à 14, **caractérisé en ce que** lesdites cellules sont impliquées dans le développement d'une structure comportant un tissu conjonctif vascularisé.

16. Procédé selon la revendication 15, **caractérisé en ce que** ledit tissu conjonctif est au moins partiellement formé de cellules mésenchymateuses.

17. Utilisation d'un support biocompatible permettant l'ancrage biologique de cellules eucaryotes dans la préparation d'un implant pour induire *in vivo* le développement d'une structure comportant un tissu conjonctif vascularisé.

18. Utilisation selon la revendication 17, **caractérisée en ce que** le tissu conjonctif est formé au moins partiellement de cellules mésenchymateuses.

19. Utilisation selon l'une des revendications 17 à 18, **caractérisée en ce que** l'implant induit la formation de néo-organe.

20. Utilisation selon l'une quelconque des revendications 17 à 19, **caractérisée en ce que** le support biocompatible est susceptible de résorption in vivo.

21. Utilisation selon l'une quelconque des revendications 17 à 20, **caractérisée en ce que** le support biocompatible est à base de calcaire.

22. Utilisation selon l'une quelconque des revendications 17 à 20, **caractérisé en ce que** le support biocompatible est à base de corail, de préférence de corail à haute porosité.
